# EUROPEAN PATENT APPLICATION

(11) **EP 3 884 859 A1**
(43) Date of publication of application: **29.09.2021**
(21) Application number: 21171621.2
(22) Date of filing: 27.06.2014
(51) Int. Cl.: A61B 5/03, A61B 5/20, A61B 5/00

(54) **SENSING FOLEY CATHETER**

(30) Priority: 27.06.2013 US 201361840408 P; 16.08.2013 US 201361959144 P; 21.10.2013 US 201361893816 P
(62) Divisional of application: 14817903.9
(71) Applicant: Potrero Medical, Inc., Hayward, CA 94545 (US)
(72) Inventor: BURNETT, Daniel Rogers, San Francisco, 94127 (US); HAMILTON, Marcie, San Francisco, 94107 (US); KEENAN, Rich, Livermore, 94550 (US); SUTARIA, Saheel, San Mateo, 94401 (US); YEE, Alex, San Francisco, 94110 (US); SKIELLER, Christina, San Francisco, 94110 (US); LUXON, Evan S, Lincoln, NE 68502 (US)
(74) Representative: J A Kemp LLP

(57) **Abstract**

Sensing Foley catheter variations are described herein which comprise a pressure sensing mechanism located within a fluid chamber or at the distal end of the catheter and configured to detect fluid pressure when body fluid, such as urine, is introduced into the drainage opening of the catheter and is received within the receiving channel and impinges upon the pressure sensing mechanism.

## Description

### CROSS-REFERENCE TO RELATED APPLICATIONS

This application claims the benefit of priority to U.S. Provisional Application No. 61/840,408 filed June 27th, 2013, U.S. Provisional Application No. 61/893,816 filed October 21st, 2013, and Provisional Application No. 61/959,144 filed August 16th, 2013, each of which is incorporated herein by reference in its entirety. This application is also related to PCT/US12/028071 filed March 7th, 2012, which is incorporated herein by reference in its entirety.

### TECHNICAL FIELD OF THE INVENTION

The disclosed technology relates to the field of medical devices, in particular devices capable of sensing physiologic data based on sensors incorporated into a catheter or implant adapted to reside in any of a urinary tract, gastrointestinal tract, rectal location, pre-peritoneal or other implanted site.

### INCORPORATION BY REFERENCE

All publications and patent applications mentioned in this specification are herein incorporated by reference to the same extent as if each such individual publication or patent application were specifically and individually indicated to be so incorporated by reference.

### BACKGROUND OF THE INVENTION

The Foley catheter, named for Dr. Frederick Foley who first described a self-retaining balloon catheter in 1929, has been in use since the 1930's, in a form nearly identical to its early models. In its most basic form, a Foley catheter has proximal portion that remains outside the body, a length that traverses the urethra, and a distal end that resides in the urinary bladder. The Foley catheter is held in place by an inflatable balloon that stabilizes the device in place, and prevents inadvertent withdrawal from the bladder. A typical Foley catheter includes at least two lumens along its length; one lumen serves as a conduit that drains the bladder, and the second lumen serves as a fluid conduit that allows the balloon to be controllably inflated and deflated.

Various developments have added diagnostic functionality to Foley type catheters, including the ability to measure pressure and temperature. For example, U.S. Patent No. 5,389,217 of Singer discloses a catheter with oxygen sensing capability. U.S. Patent No. 5,916,153 of Rhea and U.S. Patent No. 6,434,418 of Neal both disclose a pressure sensor associated with a Foley type catheter. U.S. Patent 6,602,243 to Noda discloses a temperature sensor associated with a Foley type catheter.

The Foley catheter, widespread in use, having a low cost, and easily put in place by health care professionals may offer still further opportunity as a vehicle for deriving critical diagnostic information. The technology disclosed herein provides for the delivery of highly resolved and previously unavailable diagnostic information, as may be derived from a Foley catheter with pressure sensing capability.

### SUMMARY OF THE INVENTION

The disclosed technology relates to a Foley type catheter for sensing physiologic data from the urinary tract of a patient, the physiologic data particularly including those gathered by high fidelity pressure sensing and transduction into signals suitable for processing. In some embodiments, the pressure-sensing Foley type catheter may further be enabled to sense temperature and analytes of clinical significance.

Generally, one variation of a fluid pressure sensing assembly may comprise a catheter (such as a Foley type catheter) having a length and an expandable retention member located near or at a distal end of the catheter, the catheter defining a drainage lumen at least partially through the catheter length such that a distal end of the drainage lumen terminates at a drainage opening defined near or at the distal end of the catheter, a fluid chamber defining a receiving channel and a port fluidly coupled to the drainage lumen such that the receiving channel is in fluid communication with the drainage opening, and a pressure sensing mechanism located within the fluid chamber, wherein a fluid introduced into the drainage opening is received within the receiving channel and impinges upon the pressure sensing mechanism.

In use, the catheter may be positioned within a body lumen (as further described here) and a fluid from the body lumen may be introduced through the drainage opening and into the drainage lumen. The fluid may be received through a port fluidly coupled to the drainage lumen and into a receiving channel of a fluid chamber which is positioned external to the body lumen and the fluid pressure may be detected from the fluid impinging upon a pressure sensing mechanism located within the fluid chamber.

In another embodiment of the pressure sensing apparatus, a pressure sensing catheter having a pressure sensing mechanism may be located near or at a distal end of the pressure sensing catheter, wherein the pressure sensing catheter has a diameter sized for insertion within the drainage lumen. In this variation, the pressure sensing catheter may be positioned within the drainage lumen and detect the fluid pressure when the fluid from the body lumen is introduced through the drainage opening and into the drainage lumen.

Embodiments of the disclosed technology include an air handling system. Such embodiments may be configured for autopriming of the balloon. Embodiments may further include features that prevent clogging by an air bubble and/or water droplet prevention. Water droplet prevention feature may include a hydrophilic fiber. Embodiments may further include a detection and warning system to alert for the presence of a clog, air bubble or water.

Embodiments of the Foley type catheter include a pressure sensor having a pressure interface disposed at a distal end of the catheter, a pressure transducer at a proximal end of the catheter, and a fluid column disposed between the pressure interface and the pressure transducer. When an embodiment of catheter is appropriately or functionally inserted into the urinary tract of a patient and the distal end is residing in the bladder, the pressure transducer can transduce pressure impinging on it from the pressure interface into a chronological pressure profile. The pressure profile has sufficient resolution to be processed into one or more distinct physiologic pressure profiles, including peritoneal pressure, respiratory rate, and cardiac rate.

In some particular embodiments of the Foley type catheter, the pressure profile generated by the pressure sensor has sufficient resolution such that, when sampled by a transducer at a frequency of at least about 1 Hz, it can be processed to yield a relative pulmonary tidal volume profile. In still further embodiments of the Foley type catheter, the pressure profile generated by the pressure sensor has sufficient resolution such that, when sampled by a transducer at a frequency of at least about 5 Hz, it can be processed to yield physiologic pressure profiles selected from a group consisting of cardiac output, relative cardiac output, and absolute cardiac stroke volume.

In various embodiments of the catheter, the fluid within the fluid column may include a gas, such as air or carbon dioxide, or it may include a liquid. In some embodiments wherein the fluid column includes a liquid, such liquid may include urine, as sourced from the bladder.

In various embodiments of the catheter, the pressure interface may include an elastic membrane or a substantially inelastic membrane. In some embodiments, the pressure interface is substantially homogeneous across its surface area. In other embodiments, the pressure interface can be heterogeneous, having regions that vary in composition or thickness, or having features that provide an elasticity bias.

In particular embodiments of the catheter, the pressure interface includes an expandable balloon. Such an expandable balloon may include either an elastic membrane or a substantially inelastic membrane. Embodiments of the balloon, particularly those having an inelastic membrane, upon expansion, the balloon has a volume in the range of about 0.1 cc to about 2 cc. Other embodiments of the balloon, upon expansion, may have larger volumes, for example, in a range of about 2 cc to about 5 cc, or in a range of about 5cc to about 250 cc, a volume that is greater than 250 cc. In another aspect, upon inflation, embodiments of the balloon may have a diameter that ranges between about 6mm and 8mm.

In various embodiments of the catheter, the pressure interface includes a membrane arranged across an opening. In such embodiments, the membrane is sufficiently elastic to respond to an internal-external pressure differential across its surface.

In some embodiments, the Foley type catheter further includes a temperature sensor to monitor a body core temperature of the patient. In these embodiments, the physiologic data from the temperature sensor in the system may be used to monitor body temperature and to feedback control delivery of a hypothermic treatment regimen. Temperatures sensors appropriate for the Foley type catheter may be of any conventional type, including by way of example, a thermistor, a thermocouple, or an optical temperature sensor.

In some embodiments, the Foley type catheter further includes one or more analyte sensors. Analyte sensors included in the scope of the disclosed technology include sensors for analytes of any clinical significance. For broad examples, such analytes may include any analyte selected from a group including pH, a gas, an electrolyte, a metabolic substrate, a metabolite, an enzyme, or a hormone. By way of particular examples, such analyte sensor may be able to sense any of a metabolic substrate or a metabolite, the analytes may include glucose or lactic acid. By way of example of a hormone, the analyte may include cortisol.

In some embodiments, the Foley type catheter further includes one or more electrodes arranged as electrical activity sensors. Such electrical activity sensors may deliver physiologic data that can be transformed to yield an electrocardiogram (EKG) or an electrogastrogram (EGG).

In some embodiments, the Foley type catheter further includes a light source and a light sensor, the sensor configured to capture light emitted from the light source. In some embodiments, by way of example, the light source and the light sensor may be configured to operate as a pulse oximeter, the light sensor being able to deliver a signal that can be transduced into a pulse rate. In another example, the light source and the light sensor may be configured to operate as an analyte sensor.

Some embodiments of the Foley type catheter may further include an expandable pressure-delivery balloon disposed on the catheter so as, upon expansion, to contact a wall of the bladder or the urethra; and a light source and a light sensor disposed proximate the tissue-compressing balloon. The pressure delivery balloon, the light source, and the light sensor may be arranged such that when the expandable pressure balloon is expanded so as to blanche a tissue surrounding it as detected by the light sensor, a light-based signal from the light sensor may be processed to yield a perfusion pressure on a urinary bladder wall or a urethra.

Some embodiments of the disclosed technology relate to a Foley type catheter for sensing pressure-based physiologic data from the urinary tract of a patient having a pressure sensor that includes a pressure interface and a transducer, the sensor not including a pressure-transmitting column. These embodiments typically have a pressure sensing mechanism or transducer proximate the pressure interface. Such pressure sensors may include, by way of example, any of a piezoelectric electric mechanism, an optical sensing mechanism, a microelectricalmechanical (MEMS) mechanism, or an acoustic wave sensing mechanism. When the catheter is appropriately or functionally inserted into the urinary tract and the distal end is residing in the bladder, the pressure sensor can transduce pressure impinging on it from the pressure interface into a chronological pressure profile, the pressure profile having sufficient resolution to allow differentiation into one or more physiologic pressure profiles selected from the group consisting of peritoneal pressure, respiratory rate, and cardiac rate.

The disclosed technology relates to a Foley type catheter for sensing pressure-based physiologic data from the urinary tract of a patient, as summarized above, but further being enabled to sense a physiologic response to the delivery of pressure, and thereby to determine tissue perfusion pressures. Embodiments of the Foley type catheter include a pressure sensor having a pressure interface disposed at a distal end of the catheter, a pressure transducer at a proximal end of the catheter, and a fluid column disposed between the pressure interface and the pressure transducer. Embodiments of this type further include an expandable pressure-delivery balloon disposed on the catheter so as, upon expansion, to contact a wall of the bladder or the urethra, and a light source and a light sensor disposed proximate the tissue-compressing balloon. When an embodiment of catheter is appropriately or functionally inserted into the urinary tract with the distal end residing in the bladder, the pressure transducer can transduce pressure impinging on it from the pressure interface into a chronological pressure profile. The pressure profile has sufficient resolution to be processed into one or more distinct physiologic pressure profiles, including peritoneal pressure, respiratory rate, and cardiac rate. And when the expandable pressure balloon is expanded so as to blanche a tissue surrounding it (as detected by the light sensor), a light-based signal emanating from the light sensor may be processed to yield a perfusion pressure on a urinary bladder wall or a urethra.

The disclosed technology further relates to a system for sensing and processing physiologic data from the urinary tract of a patient, the physiologic data particularly including those gathered by high fidelity pressure sensing and transduction into signals suitable for processing; these embodiments will now be summarized. In some embodiments, the pressure-sensing Foley type system may further be enabled to sense and process temperature data and/or analyte data of clinical significance; these features and embodiments will be summarized further, below.

Thus, particular embodiments of the disclosed technology relate to a system for sensing pressure-based physiologic data from the urinary tract of a patient. Embodiments of the system include a Foley type catheter with a pressure sensor having a pressure interface disposed at a distal end of the catheter, a pressure transducer at a proximal end of the catheter, and a fluid column disposed between the pressure interface and the pressure transducer. When the catheter is appropriately or functionally inserted into the urinary tract and the distal end is residing in the bladder, the pressure transducer can transduce pressure impinging on it from the pressure interface into a chronological pressure profile. Embodiments of the system further include a data processing apparatus in communication with the pressure transducer so as to be able to acquire the physiological data. Embodiments of the data processing apparatus are configured to process the chronological pressure profile into one or more physiologic pressure profiles from the group including peritoneal pressure, respiratory rate, and cardiac rate.

In particular embodiments of the system, the pressure transducer is operable to sample pressure impinging on it at a rate of at least about 1 Hz. In embodiments such as these, the data processing apparatus may be configured to determine relative pulmonary tidal volume. In other particular embodiments of the system, the pressure transducer is operable to sample pressure impinging on it at a rate of at least about 5 Hz. In embodiments such as these, the data processing apparatus may be configured to determine any of cardiac output, relative cardiac output, or absolute cardiac stroke volume.

In particular embodiments of the system, the Foley type catheter may further include a temperature sensor to monitor body temperature. In embodiments such as these, the data processing apparatus may be further configured to acquire and process signals from temperature sensor.

In other embodiments of the system, the Foley type catheter may further include one or more analyte sensors. In embodiments such as these, the data processing apparatus is further configured to acquire and process signals from the one or more analyte sensors.

In some embodiments of the system, the data processing apparatus includes a stand-alone console. In some embodiments, the stand-alone console includes a bedside unit that is dedicated to monitoring a single patient In some of these types of embodiments, the communication between the pressure transducer and the data processing apparatus is wireless.

In some embodiments of the system, the data processing apparatus includes a networked computer. In some of these types of embodiments, the networked computer is able to track data from a plurality of patients.

In particular embodiments of the system, the data processing apparatus may include both a stand-alone console and a networked computer. In some of these types of embodiments of this type, the stand-alone console and the networked computer are in communication with each other. In particular embodiments, the in communication between the stand-alone console and the networked computer is wireless.

In some embodiments of the system, the data processing apparatus may include a memory into which a normal range of values for the physiologic data may be entered, and the data processing apparatus may be configured to initiate an alarm when physiologic data of the patient are outside such range of normal values.

In some embodiments of the system, the data processing apparatus may include a memory configured to receive patient-specific clinical data from a source external to the Foley type catheter, and the data processing apparatus may be configured to integrate such external data and the Foley type catheter-derived physiologic data.

Some embodiments of the system may include a controller in communication with the data processing apparatus. In such embodiments, the controller may be configured to tune a level of pressure being applied through the fluid column against the proximal side of the pressure interface. Aspects of tuning the pressure level being applied distally against the pressure interface are expanded on below, in the context of summarizing methods provided by the disclosure. Further, in embodiments of the catheter that include a pressure delivery balloon that may be used in a method to measure tissue perfusion pressure, the controller may be configured to controllably expand such pressure delivery balloon.

In some embodiments of the system, the physiologic data from the pressure sensor may be used to track clinical parameters relevant to monitoring intraabdominal hypertension (IAH) or abdominal compartment syndrome (ACS). In other embodiments of the system, the physiologic data from the pressure sensor may be used to track clinical parameters relevant any of monitoring cardiac status, respiratory status, the onset and progression of hemorrhage or shock, patient bodily movement, or intestinal peristalsis.

As noted above, some embodiments of the disclosed technology relate to a system for sensing pressure-based and temperature-based physiologic data from the urinary tract of a patient, such system including a Foley type catheter with a pressure sensor and a temperature sensor. Embodiments of the pressure sensor have a pressure interface disposed at a distal end of the catheter, a pressure transducer at a proximal end of the catheter, and a fluid column disposed between the pressure interface and the pressure transducer. When the catheter is appropriately or functionally inserted into the urinary tract and the distal end is residing in the bladder, the pressure transducer transduces pressure impinging on it from the fluid column into physiological data comprising a chronological pressure profile. Embodiments of the system further include a data processing apparatus in communication with the pressure transducer so as to be able to acquire the physiological data. Embodiments of the data processing apparatus are configured to process the chronological pressure profile into one or more physiologic pressure profiles from the group including peritoneal pressure, respiratory rate, and cardiac rate. Embodiments of the data processing apparatus are further configured to acquire and process signals from the temperature sensor, such signals reporting the core body temperature of the patient.

Some embodiments of the disclosed technology relate to a system for sensing pressure-based and analyte-based physiologic data from the urinary tract of a patient, such system including a Foley type catheter with a pressure sensor and one or more analyte sensors. Embodiments of the pressure sensor have a pressure interface disposed at a distal end of the catheter, a pressure transducer at a proximal end of the catheter, and a fluid column disposed between the pressure interface and the pressure transducer. When the catheter is appropriately or functionally inserted into the urinary tract and the distal end is residing in the bladder, the pressure transducer transduces pressure impinging on it from the fluid column into physiological data comprising a chronological pressure profile. Embodiments of the system further include a data processing apparatus in communication with the pressure transducer so as to be able to acquire the physiological data. Embodiments of the data processing apparatus are configured to process the chronological pressure profile into one or more physiologic pressure profiles from the group including peritoneal pressure, respiratory rate, and cardiac rate. Embodiments of the data processing apparatus are further configured to acquire and process analyte signals from the one or more analyte sensors, such signals reporting the level of one or more analytes within the urinary tract.

As noted above, some embodiments of the disclosed technology relate to a system for sensing pressure-based, temperature-based, and analyte-based physiologic data from the urinary tract of a patient, such system including a Foley type catheter with a pressure sensor, a temperature sensor, and one or more analyte sensors. Embodiments of the pressure sensor have a pressure interface disposed at a distal end of the catheter, a pressure transducer at a proximal end of the catheter, and a fluid column disposed between the pressure interface and the pressure transducer. When the catheter is appropriately or functionally inserted into the urinary tract and the distal end is residing in the bladder, the pressure transducer transduces pressure impinging on it from the fluid column into physiological data comprising a chronological pressure profile. Embodiments of the system further include a data processing apparatus in communication with the pressure transducer so as to be able to acquire the physiological data. Embodiments of the data processing apparatus are configured to process the chronological pressure profile into one or more physiologic pressure profiles from the group including peritoneal pressure, respiratory rate, and cardiac rate. Embodiments of the data processing apparatus are further configured to acquire and process signals from the temperature sensor, such signals reporting the core body temperature of the patient. Embodiments of the data processing apparatus are further configured to acquire and process analyte signals from the one or more analyte sensors, such signals reporting the level of one or more analytes within the urinary tract.

In some embodiments of the system, the physiologic data from the any one or more of the sensors (pressure sensor, temperature sensor, and/ or analyte sensor) may be used to track clinical parameters particularly relevant to monitoring clinical conditions brought about by metabolic diseases or diseases with pathophysiologic metabolic symptoms. For example, embodiments of the system may be used to monitor clinical parameters relevant to kidney function or diabetes. In other embodiments of the method, the physiologic data from the sensors, the pressure sensor in particular, may be used to monitor body movement.

Some embodiments of the system include a fluid-collecting receptacle to collect urine drained from the bladder, and the receptacle may include a fluid volume measuring system. In some of such embodiments, the fluid volume measuring system is configured to deliver data from which a urine output rate may be determined. Embodiments of the fluid volume measuring systems may include any of a weight-sensitive system, a fluid height sensing system, a mechanical mechanism, or an optically-sensitive system.

Some embodiments of the fluid-collecting receptacle may include a chemical analyte measuring system to identify and/or quantitate analytes such as those summarized for the Foley type catheter itself. More specifically, as example, analyte sensors may be sensitive to any one or more analytes selected from a group consisting of bacteria, blood, hemoglobin, leukocyte esterase, glucose, and particulate matter.

Some embodiments of the fluid-collecting receptacle may include an RFID chip for identification of the receptacle in communications with a data processing apparatus, or for conveying sensed data to the data processing apparatus.

Some embodiments of the system may include a docking station to accommodate the collecting receptacle, wherein the docking station and the collecting receptacle are in electrical communication with each other. Communication between the docking station and the collecting receptacle may occur by way of a data transmission line connecting the docking station to the console, or it may occur by way of a wireless communication system.

Some embodiments of the system may include a fluid infusion apparatus, with the data processing apparatus being configured to control the activity of the fluid infusion apparatus in response to physiologic data processed by the data processing apparatus.

Some embodiments of the disclosed technology relate to a method for monitoring physiologic data from the urinary tract of a patient. These physiologic data particularly include pressure-based data, but may further include temperature-based data and analyte-based data. In still further embodiments, delivery of pressure in combination with light-based data to yield tissue perfusion pressure values.

Embodiments of the method include providing a physiologic data monitoring system that includes a Foley type catheter and a data processing apparatus. Embodiments of the Foley type catheter have a pressure sensor, the pressure sensor having a pressure interface disposed at a distal end of the catheter, a pressure transducer at a proximal end of the catheter, and a fluid column disposed between the pressure interface and the pressure transducer, the pressure transducer being able to transduce pressure impinging on it from the fluid column into physiological data comprising a chronological pressure profile. The method may further include inserting the Foley type catheter in the urinary tract such that the pressure interface is residing within the patient's bladder; transferring pressure sensed in the bladder into a transducible chronological pressure profile; and processing the chronological pressure profile into one or more physiologic pressure profiles selected from the group consisting of peritoneal pressure, respiratory rate, and cardiac rate.

Some embodiments of the method include tuning or priming a level of pressure being applied from a proximal side of the pressure interface of a Foley type catheter toward equivalence with a baseline physiologic pressure being applied to a distal side of the pressure interface. Tuning pressure refers generally to either increasing or decreasing pressure applied to the proximal side of the pressure interface. Proximal, in this context, refers to the side of the pressure interface facing outward from the body (within the communicating fluid column), and toward the main body of the catheter or an operator handling the catheter. In one aspect, tuning the pressure level may refer to priming the fluid column from the proximal end of the column, directing pressure toward the distal end of the column. In another aspect, tuning the pressure level may refer to releasing or bleeding pressure from the proximal end of the column, as may be appropriate, for example, if pressure in the column overshoots a desired pressure level, or if pressure from within the bladder were to decrease. Embodiments of the method may further include repeating the tuning step, as needed, to maintain equivalence between the level of pressure being applied from the proximal side of the pressure interface and the baseline physiologic pressure being applied to a distal side of the pressure interface.

Embodiments of the tuning step of the method may include monitoring a physiologic pressure profile, and adjusting the pressure being applied from a proximal side of the pressure interface to a level such that a quality of a physiologic pressure profile being processed by the system is optimized. By way of example, the amplitude of pressure waves associated with the respiratory rate may be monitored. A high amplitude pressure profile may be considered optimal in that it is generally associated with conditions of equivalence between baseline pressure on either side of the pressure interface. In another aspect, a high amplitude pressure profile may be considered optimal because, other factors being equal, a high amplitude signal permits a higher level of resolution of real differences that may appear in signal level. In some embodiments, the monitoring step may be performed automatically by the data processor, and the adjusting step may be performed by an automatic controller in communication with the data processor.

The desire to prime the catheter is driven, at least in part, by leakage of gas from the fluid column. It has been observed, for example, that a Foley type catheter, per embodiments of the disclosed technology, that comprises a thin silicone membrane (e.g., a membrane with a thickness of 0.003 inch) leak about 2cc of air per hour when under 15mm Hg of pressure.

Some embodiments of the method may include applying pressure to the proximal side of the pressure interface by delivering gas under pressure a space proximal to the pressure interface. Delivering gas to the space proximal the pressure interface may be considered priming the space or tuning the space so as to equilibrate or substantially equilibrate pressure on either side of the pressure interface. The source of the gas, per embodiments of the technology, may be a compressed gas cylinder, or may be a pump using atmospheric air or other fluid. Any suitable biologically compatible gas may be used, including, by way of example, air or carbon dioxide.

In some embodiments of the method, appropriate for those in which the pressure interface includes a balloon formed from an inelastic membrane, the method further includes priming the fluid column from the proximal end of the catheter to maintain the balloon at a size that places no substantial strain on the inelastic membrane.

In some embodiments of the method, appropriate for those in which the pressure interface includes a balloon formed from an inelastic membrane having a total surface area, the method further include inflating the balloon to a level such that the total surface area of the membrane is substantially taut.

Some embodiments of the method include sampling the pressure profile impinging on the transducer at a frequency of at least 1 Hz, the method further comprising quantifying respiratory excursions relative to a baseline magnitude of excursions proximate the time of catheter insertion. These embodiments may particularly include monitoring the relative amplitude of respiratory pressure wave excursions, and relating such relative amplitude to relative respiratory tidal volumes.

Some embodiments of the method include sampling the pressure profile impinging on the transducer at a frequency of at least 5 Hz, the method further including quantifying peaks on the respiratory pressure wave that are associated with the cardiac rate. In particular embodiments of this type, against a background of a substantially stable peritoneal pressure, the method may further include determining any of cardiac output, relative cardiac output, respiratory tidal volume, or absolute cardiac stroke volume.

In some embodiments of the method, the one or more physiologic pressure profiles yielded by processing the chronological pressure profile may provide for monitoring of body movement Monitoring body movement may be of particular benefit for bed-ridden patients, for example, who have a decubitis ulcer, or are at risk of developing such an ulcer when a portion of the body, such as a bony prominence, rests too long in a pressured position without movement that would relieve such pressure. Accordingly, monitoring body movement may include notifying a health care provider of the level of movement of a patient who is at risk of developing a decubitis ulcer, or at risk of exacerbating an existing decubitis ulcer. In addition, monitoring of patient activity may also affirmatively report the presence of movement. In this case, a patient that is a fall risk can be monitored for activity that may indicate an attempt to rise from their bed. This may signal an alert and prevent their mobility without assistance.

In some embodiments of the method, wherein the Foley type catheter has an expandable pressure delivery balloon, a light source and a light sensor proximate the expandable pressure balloon (the light sensor configured to capture light from the light source) the method may further include inflating the pressure delivery balloon to a desired pressure, and monitoring the pressure within the expandable balloon to determine the pressure level required to blanche the tissue, said blanching pressure being reflective of a tissue perfusion pressure.

In some embodiments of the method, wherein the Foley type catheter has a temperature sensor, the method may further include monitoring the body temperature of the patient In some embodiments of the method, wherein the Foley type catheter further comprises an analyte sensor, the method further may further include monitoring a level of the analyte within the urine of the patient.

Embodiments of the disclosed technology include a method of mining data from pressure/acoustic signal. Such data may include values for parameters such as intraabdominal pressure, heart rate and stroke volume/cardiac output, respiratory rate and tidal volume, bowel activity, patient movement detection, behavioral compliance (periodic movement and/or immobility), seizure or shivering detection, cough frequency and severity, speech detection, and sleep duration and sleep quality. Dehydration may also be determined by monitoring respiratory rate, heart rate, blood pressure, temperature etc. Internal bleeding may also be determined by detecting increases in intraabdominal pressure. Blood volume changes as low as 50cc or lower may be able to be detected.

Embodiments of the disclosed technology can determine the effectiveness of chest compressions during CPR or other lifesaving activities.

Embodiments of the disclosed technology may include product expiration technologies so that the products are not used for too long a period or re-used if disposable. For example, products may include a mechanical or electrical kill switch, which may be based on time frame, time frame from initial use, number of uses etc. Products may also be labeled with Radio-frequency identification (RFID) to prevent reuse. In some embodiments the controller reports and/or displays how long the catheter has been in use.

Embodiments of the disclosed technology may be configured for automation of feedback to control another device. Such automated aspects may include ventilator settings based on intraabdominal pressure (IAP), IV fluid infusion based on based on IAP, pressure-based diagnostics, drug delivery i.e., shivering prevention, paralytics, etc., temperature control as may be applied to fever prevention or therapeutic hypothermia, triggering urine flow with increased bladder pressure (which may be advantageous for allowing for natural downstream sweeping of bacteria and for reducing risk of infection), base station alerts with centralized reporting and data collection and synchronization with mobile alerts, and signal analysis and/or predictive algorithms to provide useful clinical data from sensors.

Embodiments of the disclosed technology may be configured for sensing in urine or on urinary tissues such as the urethral mucosa. Sensing capabilities to be applied to the urethral mucosa may include pH, microdialysis, pyruvate, lactate, pO2, pCO2, perfusion index, near- infrared spectroscopy, laser Doppler flowmetry, urethral capnography, and orthogonal polarization spectroscopy, temperature, pulse oximetry, perfusion pressure, detection and prevention of infection, and detection of analytes that are informative regarding health status of the patient such as (merely by way of example) procalcitonin, lactoferrin, leukocyte esterase, specific gravity, pH, protein, glucose, ketones, blood, leukocyte esterase, nitrite, bilirubin, urobilinogen, ascorbic acid.

Embodiments of the disclosed technology include a device for sensing in the bladder or urethra, wherein the device may sense any one or more of temperature, acoustic detection of body sounds and sound transmission (such as those that may occur during speech, apnea, sleep apnea, respiratory wheezes/rhonchi, pneumonia, asthma, ARDS, cardiac tamponade, murmur), pulse oximetry, perfusion pressure, electrocardiogram, electromyogram, or pressure.

Various embodiments may be applied to any cavity or lumen (GI, urinary, gynecologic). Embodiments may further include implantable sensors (pre-peritoneal, bladder wall, etc.) and free floating sensors (GI tract, bladder, etc.). Pressure sensors included within the scope of the disclosed technology may be of any conventional type, such as those configured for air, fluid, or solid state transmission. Embodiments of the technology may include a battery backup that allows travel with patient. Embodiments may include a controller with its own display and alerts.

Embodiments of the disclosed technology include embodiments where the retention balloon is only slightly inflated in order to increase balloon sensitivity to small changes in pressure. This may allow for finer measurements of micro parameters, such as heart rate, relative stroke volume, relative cardiac output, respiratory rate, and relative tidal volume.

Embodiments of the disclosed technology include a fully implantable device or a device fully enclosed in a luminal site (temporary or long-term) and may be used to sense any of the parameters disclosed above, and report these parameters externally to provide diagnostic information to the healthcare provider. Implantable embodiments may be enabled with pressure sensing capability as well as one or more analyte sensing capabilities, and further may be enabled with data processing capabilities to yield values for various physiologic parameters, as has been described herein, in the context of the sensing Foley catheter embodiments.

Implantable embodiments may employ a balloon positioned in the pre-peritoneal space. The balloon may be in fluid communication with a pressure sensor within the device and the pressure reported, intermittently or continuously, externally. The implantable device may also be rechargeable and may report any parameters mentioned herein. In particular, the implantable device, or an external controller, may be capable of extracting information from the pressure signal to give an indicator of respiratory rate, cardiac rate and/or relative cardiac output or relative stroke volume. The implantable device may be placed fully within the preperitoneal space or may be partially or fully placed within the subcutaneous space. The device may be recharged transdermally, possibly in its preperitoneal site or via a tethered antenna implanted closer to the skin. The device may have its battery changed once every few years or may be inductively powered or recharged by a custom belt that may be worn over the device for all or part of the day. The device may have therapeutic abilities and be able to perform an action based on sensed parameters. In addition to calling help, the device may be able to deliver a shock in response to changes in cardiac output, stroke volume, and/or heart rate sensed by the device or deliver a drug in response to any changes in the sensed parameters. The device may also communicate with the patient through a receiver or smart phone which may allow for automatic uploading of data to a healthcare provider. The device can be implanted anywhere in the body. In a preferred embodiment, for optimal acoustic and pressure data, the device may be placed in the pre-peritoneal space superior to the umbilicus just below the xiphoid. This embodiment may measure respiratory rate, cardiac rate, relative cardiac output, relative stroke volume, patient activity level, or peristaltic activity and data processing by way of algorithms may be applied to yield clinically applicable information. By applying the algorithms of this present technology (for example, by selectively filtering the noise, extracting frequencies, or reporting certain frequencies as physiologic signals), each of these parameters may be obtained from the peritoneal pressure signal.

Other body sounds, such as bowel sounds, heart sounds, and respiratory sounds may also be transmitted and detected in order to detect pathology related to changes in these sounds (for example, bowel obstruction, pneumonia, or decreased cardiac output). In some embodiments, the device has adequate hoop strength to support an acoustic/pressure sensing membrane to ensure that capsular contracture does not occur. In these embodiments the hoop may be constructed of nitinol to allow for its compression into a small delivery package. The preperitoneal space may be dissected using a blunt dissection tool at an angle to the peritoneal lining and the device deployed into this space by expansion into a larger configuration. In some embodiments, this design may also include a small catheter for accessing the peritoneal cavity to sense analytes within the peritoneal fluid and/or deliver compounds to this space. Implantable embodiments may be used as long-term implants monitoring chronic conditions (ie monitoring for fluid on the lungs, cardiac output, etc. for congestive heart failure, monitoring heart rate and respiratory rate for any condition that can cause acute decompensation, etc.) while allowing the patient to remain ambulatory. The implantable device may be positioned close to any organ of interest (i.e. over lower quadrants for monitoring of bowel sounds).

Embodiments of the disclosed technology include embodiments where temperature is measured and tracked over time. Also, acceleration data may be recorded and used to measure patient activity levels. Acceleration data may also be combined with other data, such as pressure and acoustic data, to more accurately identify events such as coughs or sneezes and filter out external artifacts. In other embodiments, the device may have offset electrodes to measure electrical cardiac activity. In other embodiments, the device may also have a glucose sensor that can continuously track the patient's blood glucose levels.

Embodiments of the disclosed technology include acoustic detection of body sounds and sound transmission through the use of a microphone and/or an acoustic signal generator and/or other technologies disposed within the sensing catheter or implant. Acoustic sound detection may also allow for the detection of speech, sleep apnea, sleep stage characterization, respiratory wheezes/rhonchi, pneumonia, asthma, acute respiratory distress, or other abnormal respiratory sounds, intestinal sounds, or cardiac sounds. Acoustic sound detection may also be used to detect changes in heart sounds that may occur with progression or onset of an illness (ie the third heart sound) or changes in bowel sounds that may indicate progression or onset of an illness (ie high pitched bowel sounds with bowel obstruction in high risk candidates).

Embodiments of the disclosed technology include embodiments which are able to detect indicators or markers of infection, such as, by way of example, urine nitrates, urine pH, glucose, leukocyte esterase, etc. These markers may be continuously or intermittently monitored. In these embodiments, a change in such infection markers in the urine may be detected and reported to prompt further investigation of a potential urinary tract infection and/or removal or replacement of the catheter. A catheter with this sensing capability may be able to be left in place for a longer duration for some patients, such as those considered at risk but who have not yet shown signs of infection. A shorter implantation period may be appropriate for patients who have already been diagnosed with an infection, in which case the catheter may be useful for monitoring resolution of an infection while the patient is being treated.

These embodiments allow infections to be prevented and/or treated early and have the potential to allow optimal residence time for each individual catheter versus the relatively arbitrary recommendation to remove and replace all Foley catheters after 7 days of dwell time. Urinary tract infections may also be rapidly detected and treated, thus resulting in a shorter overall hospital stay for these patients. Sensors within the catheter or within the collection reservoir may also detect urine flow rate (catheter or reservoir based), bacteria presence, procalcitonin, lactoferrin, leukocyte esterase, specific gravity, pH, protein, glucose, ketones, blood, leukocyte esterase, nitrite, bilirubin, urobilinogen, ascorbic acid. The pressure sensor may also allow for triggering of urine flow with increased bladder pressure, which mimics the natural flow of urine and sweeps bacteria downstream (and may reduce infection). In this scenario, a valve may be incorporated into the urine outflow line that may be intermittently opened and closed based on bladder pressure.

These embodiments may allow rinsing lavage of the bladder, so as to treat infection or other insult or injury to the bladder. A lavage may serve, for example, to cleanse the bladder interior of bacteria or blood clots. Further, anti-infective agents may be delivered through embodiments of the disclosed catheter.

A balloon or an infusion catheter that slowly infuses fluid may also be used to sense peritoneal or intraabdominal or other pressure through placement in peritoneal sites other than the bladder, such as the rectum or stomach. Regardless of where the sensing occurs (bladder, rectum, stomach, etc.) or whether the pressure transmission medium is liquid or air, the method of determining parameters such as respiratory rate, cardiac rate, relative cardiac output, relative stroke volume, patient activity level, or peristaltic activity, data processing by way of algorithms may be applied to yield clinically applicable information. By applying the algorithms of this present technology (for example, by selectively filtering the noise, extracting frequencies, or reporting certain frequencies as physiologic signals), each of these parameters can be obtained from this peritoneal pressure signal. Other body sounds, such as bowel sounds, heart sounds, and respiratory sounds may also be transmitted and detected in order to detect pathology related to changes in these sounds (for example, bowel obstruction, pneumonia, or decreased cardiac output).

In some embodiments, noise filtering may have requirements particular physiological pressure measurements. For example, noise in this situation may include patient coughing, moving, or other types of noise not normally found in signal filtering algorithms. Some embodiments may, for example, measure heart rate and then use this rate to determine a physiological range for acceptable heart rate. If the heart rate is measured beyond this range (either above or below it), the controller may determine that the signal is noisy and either ignore it, or apply noise filtering technology to the signal. The same method may be applied to other, somewhat predictable, signals, such as respiratory rate, respiratory pressure, IAP, etc.

Other signal filtering techniques may be used to distinguish between noise and actual signal. For example, the respiratory frequency and the heart frequency signals are generally distinct from each other. However, under certain circumstances, the frequencies may overlap. In this situation other factors may need to be considered in the pressure signal analysis algorithm, for example signal amplitude.

Some embodiments of the disclosed system may be functionally directed to the delivery of therapeutic hypothermia. In this clinical application, the catheter may be equipped to measure bladder pressure, as above, measure urethral temperature, and be able to drain urine and add fluid to the bladder. In this embodiment, the catheter may be used to warm or cool the patient (mild to moderate hyperthermia or mild to moderate hypothermia) via the infusion of a warm or cold fluid as appropriate. In the generation of mild to moderate hypothermia, the bladder may be evacuated then refilled to a set pressure with an ice-cold medium (a cold fluid, or a chilled slurry or slush) while the core body temperature is monitored. In this embodiment, an initial fill of the bladder with cold medium may be sufficient to generate the desired degree of hypothermia, or the temperature of the fluid may be tracked (in some embodiments, by way of a second temperature sensor in the bladder) and evacuated once it rises above a set temperature (e.g., 15°C). If the desired patient temperature has not yet been reached, the bladder may then be refilled with the liquid/slurry and evacuated until the patient has achieved their target temperature.

In some embodiments, the therapeutic hypothermia process is automated by the system, requiring only that a clinician insert a sensing Foley catheter embodiment, and then connecting the catheter to the temperature control system and/or any patient monitor that the clinician desires. In some embodiments, the infused fluid is a slush to take advantage of the much greater watt extraction capabilities of slush in comparison to a cold fluid. In some embodiments, the sensing Foley catheter is able to sense one or more of the other parameters mentioned above (such as respiratory rate, or oximetry) during and following this therapy. The cold medium (slush and/or fluid) may be used to induce hypothermia, and the bladder may be evacuated once the target temperature is reached. As the body temperature rises, the slush and/or fluid may be introduced into the bladder then evacuated, again, as the target temperature is reached. In this embodiment, the resting state of the bladder is the evacuated state and it only contains chilled fluid or ice when the body is not within target temperature range. In some embodiments, the slush may be formed on-demand in a manner that allows it to be carried into the field or ambulance, and then created on-site, in order to treat trauma or injury as it occurs. This on-demand aspect of the method embodiment may involve a pre-frozen block of ice that is shaved or ground, or a compressed gas source that vents into the liquid, thereby causing a rapid drop in temperature. This compressed gas embodiment may be used either to generate a slush, or to cool the medium while allowing it to remain a liquid.

A similar technique may be used with certain embodiments to induce hyperthermia with a warm or hot liquid.

Variations of the embodiments described above for use in the bladder, may be reconfigured and/or resized for application in other luminal body sites such as the stomach, esophagus, small intestine, large intestine or rectum. In some embodiments, these data may be obtained through invasive access of the peritoneal cavity, cerebrospinal space or pleural space, ideally in instances where accessing these spaces is already performed for another purpose.

Some embodiments of the device may incorporate mechanisms to keep the urine lumen, or other lumen, clear of blockages in order to maintain an empty, flaccid bladder and avoid false positive IAP measurements. These blockages may be caused by airlocks in the drainage tube or by crystals, blood clots, or other physical blockages. Any of the embodiments to keep the line clear as described in Burnett PCT/US2013/060003, herein incorporated by reference, would be suitable. In one embodiment, this is accomplished with active line clearing, such as a bellows to provide negative pressure or a pump to clear obstructions. This embodiment allows for clearing of both airlocks and physical blockages. In another embodiment, the line clearing is passive, and may be accomplished with vents that allow air to escape the drainage line instead of forming airlocks. In yet another embodiment, the IAP measurements from the present device may be combined with urine output measurements obtained with the Burnett device, in any manner they have disclosed.

Some embodiments of the disclosed technology may comprise methods of pressure measurement in other anatomic locations and/or combined with existing medical devices. In one embodiment, the pressure-sensing system of the present invention may be used with ascites shunts in order to ensure that the shunt is draining and has not become obstructed. In another embodiment, the pressure-sensing system may be used with dialysis catheters. In another embodiment, the system may be used with insulin delivery catheters. Generally, the system may be used with any shunting, infusing, or other similar applications where fluid blockage may be of a concern and a pressure measurement would help identify whether a blockage has occurred.

Embodiments of the disclosed technology may integrate with, or link to other medical system, including an Electronic Health Record (EHR), Electronic Medical Record (EMR), clinical trial software, research software, medical monitoring systems, EKG systems, infusion systems, drug delivery systems, heart rate monitor systems, body vital sign monitoring systems, respiratory rate systems, etc. For example, pressure data collected from any of the embodiments discussed herein may be imported into, or integrated with an EMR so that a physician has a full picture of a patient Any other data collected and/or analyzed by the disclosed embodiments can be used in a similar way. For example, a user may analyze clinical trial data which has been integrated with a controller incorporated into one of the disclosed embodiments. The user may view individual patient data to determine if there is any data to support abnormal heart rate, abdominal pressure, urine flow etc. Integration with an EHR may be done via a standard web browser using html and frames/windows/window areas, or XML or using any other appropriate standard or technology.

Data from disclosed embodiments, either alone, or in conjunction with data from integrated systems, may be stored, tracked and/or mined. The disclosed systems may "learn" from the stored data in such a way to provide recommendations on treatment or diagnoses. Systems may be networked so that data from more than one patient can be aggregated and used for this purpose. For example, embodiments of the disclosed technology may analyze data from multiple patients who have an elevated respiration rate, an elevated heart rate, and/or increased intraabdominal pressure. By analyzing data from these patients in conjunction with data from the EHR, embodiments of the disclosed technology may be able to determine that patients with this data profile, are more likely to have a particular disease and may therefor recommend a blood test, or may automatically perform a urine analyte test.

In the same way, an upward trending temperature in conjunction with one or more other measured parameters may be an indication of infection. Additional tests, or an infusion, may be recommended or performed on the patient automatically or with user confirmation.

Data may also be tracked to determine the time until obstruction and/or infection for one patient, or across multiple patients.

Embodiments of the technology include a sterile to non-sterile attachment between the catheter device and the pressure transducer. Since the catheter may be sterile and disposable and the pressure transducer may not be sterile nor disposable, it is important to be able to connect the two components without increasing the risk of infection to the patient Filter paper, such as 0.2 micron filter paper, or other suitable material, may cover the portion of the catheter where the pressure transducer connects to the catheter.

Embodiments of the technology may include a pressure sensor and logic to manage the balloon inflation of the retention balloon in addition to the pressure balloon. In some embodiments the retention balloon can serve as both a retention balloon and a pressure balloon, this may be particularly applicable when only IAP is being measured. In other embodiments, the retention balloon can sense pressure and the logic of the controller can detect when the pressure of the retention balloon falls outside expected ranges, and may alert the user in some way, such as an alarm. For example, if the catheter is tugged, or the patient tries to remove it, the pressure in the retention balloon will increase. This increase in pressure could be programmed to sound an alarm. In another example, a technician may attempt to inflate the retention balloon before the catheter tip is fully placed within the bladder. In this case, if the retention balloon were inflated in the urethra, the pressure would be higher than normal and an alarm or other alert could result. Acceptable retention balloon pressure ranges may be determined by tracking retention balloon pressures across several patients to determine the normal range of pressures. Pressures outside of this range may be programed to send/sound an alert, or to automatically reduce the balloon pressure.

Pressure sensing can also be used in either the retention balloon or pressure balloon to detect bladder spasms. A sudden, or repeated, change in pressure could be an indication of bladder spasm. The controller may be programmed to send an alert, or to change the pressure of the balloon when an apparent bladder spasm is occurring.

Embodiments of the technology may include acoustic sensing to determine the size and/or volume of the bladder. This technology may be useful in determining the air in the bladder, or the Gastric Residual Volume (GRV). Bladder size may be measured by creating and sensing acoustic waves and determining the time between wave emission and wave sensing after the wave has bounced off of the bladder wall. This measurement may be performed at one or more than one location within the bladder.

Another method of measuring bladder volume includes measuring the temperature change within the bladder using an embodiment of the present invention after introduction of a cool or warm fluid. The time it takes to warm or cool the fluid in the bladder is related to the bladder volume.

Embodiments of the technology may include self cleaning technologies. For example, a Foley catheter system may be automatically flushed with saline. A Foley catheter may also be purged by using natural bladder pressure, or by various pumping/pressure mechanisms disclosed herein.

Embodiments of the technology may include the ability to detect deficient connections within the system. For example, mechanical sensors may detect integrity of the connections between any components of the system. Alternatively, connection integrity may be sensed through small pressure changes, or other pressure sensors.

Embodiments of the technology may include alternative materials for the Foley catheter system. For example, the catheter shaft, or part of the catheter shaft, may include an outer, inner or embedded braid or other more rigid material to prevent the catheter from kinking. For example, the pressure lumen may have a more rigid inner surface, such as a polymer, braid etc. The added rigidity may also increase the sensitivity of pressure measurements through the lumen.

Embodiments of the technology include an implantable sensor for vital sign monitoring, as particularly suitable for a patient in battlefield or transport setting, prior to being secured into a hospital setting.

Embodiments of the technology include a free-floating transmitting bladder embodiment. Embodiments of the technology include a free-floating transmitting stomach embodiment. Embodiments of the technology include an ingestible, self-destructing capsule. Embodiments of the technology include vagina, stomach, intestine, esophagus, or a rectum sensor.

Embodiments of the technology include a catheter for sensing physiological data from a urinary tract of a patient comprising a pressure sensor comprising a pressure interface disposed at a distal end of the catheter, a first pressure transducer at a proximal end of the catheter, and a first fluid column disposed between the pressure interface and the first pressure transducer, a second pressure transducer at the proximal end of the catheter and a second fluid column disposed between the pressure interface and the second pressure transducer, wherein, when the catheter is inserted into the urinary tract and the distal end is residing in the bladder, the first pressure transducer can transduce pressure impinging on it from the pressure interface into a first chronological pressure profile, and the second pressure transducer can transduce pressure impinging on it from the pressure interface into a second chronological pressure profile.

Embodiments include a catheter where the first fluid column and the second fluid column are separate fluid columns for the length of the catheter.

Embodiments include a catheter where the first fluid column and the second fluid column are separate fluid columns for part of the length of the catheter, and the same fluid column for part of the length of the catheter.

Embodiments include a catheter where the first fluid column and the second fluid column are the same fluid column for the length of the catheter.

Embodiments include a catheter where the pressure interface comprises a balloon.

Embodiments include a catheter where at least one fluid column is in communication with a physical filter.

### BRIEF DESCRIPTION OF THE DRAWINGS

Fig. 1 shows a data console in communication with a urine-collecting receptacle docking station, per an embodiment of the sensing Foley catheter system.
Fig. 2 shows an embodiment of the sensing Foley catheter system set up to measure urine output from a human subject.
Fig. 3 shows an embodiment of the sensing Foley catheter system set up as an automated infusion therapy system for a human subject.
Fig. 4 shows a volume-sensing urine collecting receptacle that may include an RFID chip, the receptacle accommodated within a receptacle docking station, per an embodiment of the sensing Foley catheter system.
Fig. 5A shows a sensing Foley catheter with a pressure interface in the form of an inflatable balloon, per an embodiment of the sensing Foley catheter system
Fig. 5B shows a sensing Foley catheter a pressure interface in the form of a membrane arranged across a luminal opening, per an embodiment of the sensing Foley catheter system.
Figs. 6A - 6D show various views and details of a sensing Foley catheter, per an embodiment of the sensing Foley catheter system.

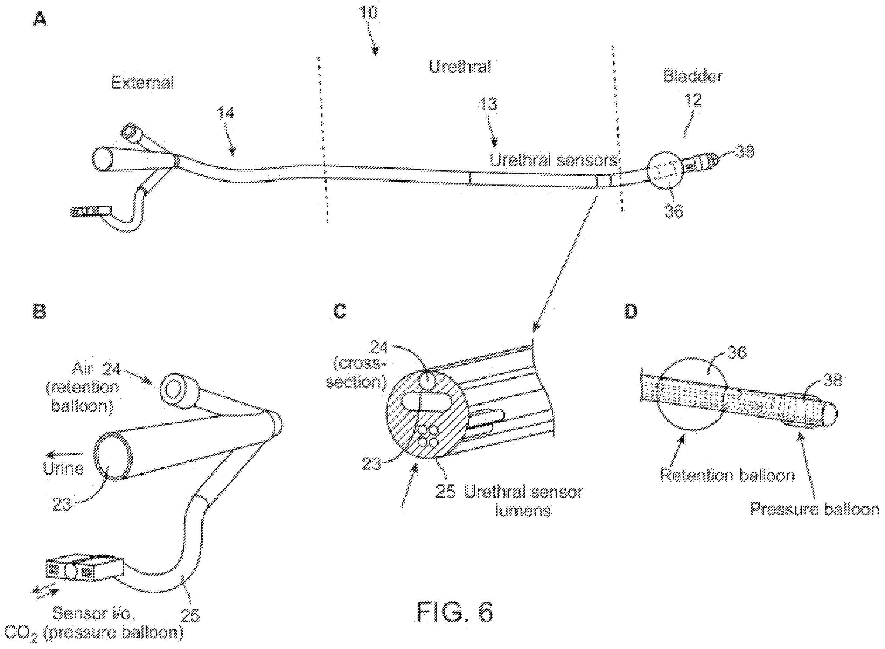

Fig. 6A schematically arranges the sensing Foley catheter into a proximal section that remains external to the body when in use, a portion that resides in the urethra, and a portion that resides in the bladder, when placed into a human subject.
Fig. 6B shows a detailed view of the proximal portion of the catheter.
Fig. 6C shows a cross sectional view of the central length of the catheter.
Fig. 6D shows a detailed view of the distal portion of the catheter that resides in the bladder.

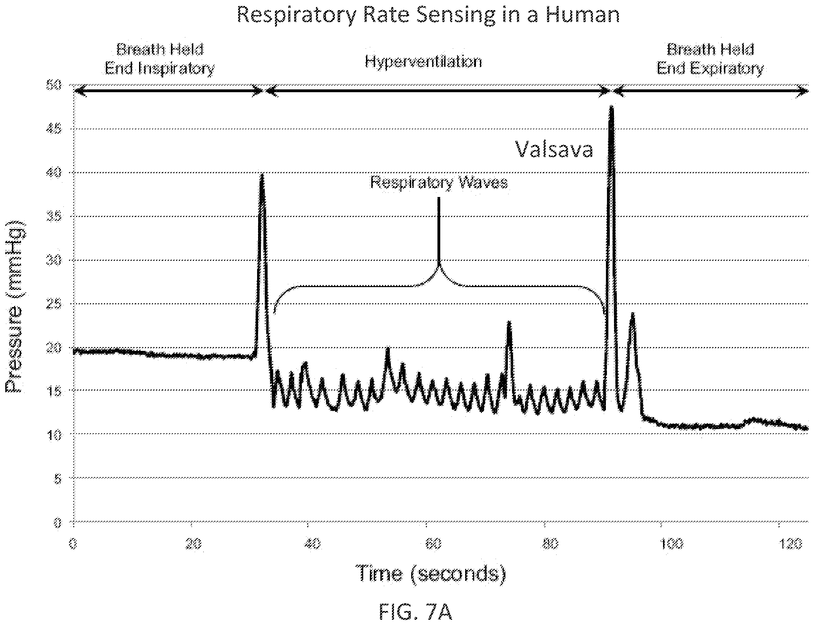

Fig. 7A shows an example of respiratory rate sensing data from a human subject, as provided by an embodiment of the sensing Foley catheter system During this test period, the subject performs a respiratory sequence as follows: (1) breath being held at the end of an expiration, (2) valsalva, (3) normal respiration, (4) valsalva, and (5) breath being held at the end of an expiration.

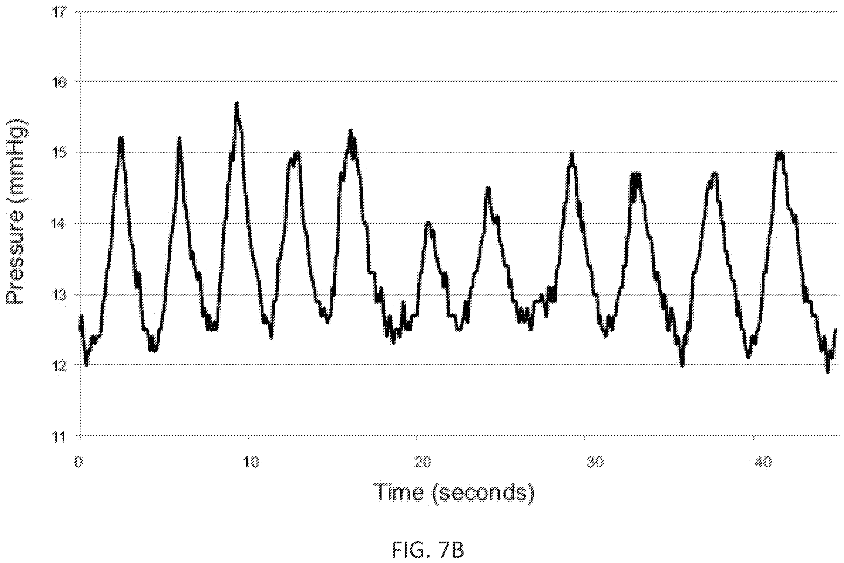

Fig. 7B shows a detailed portion of the respiratory profile of Fig. 7A, a portion of the period of normal respiration.

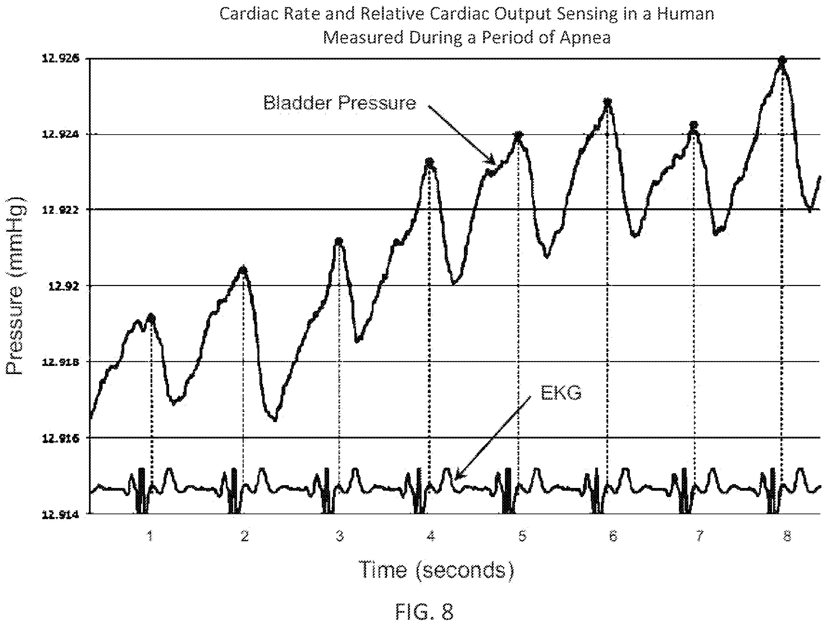

Fig. 8 shows an example of cardiac rate and relative cardiac output sensing data from a human subject, as provided by an embodiment of the sensing Foley catheter system, and an EKG trace as measured simultaneously and independently.

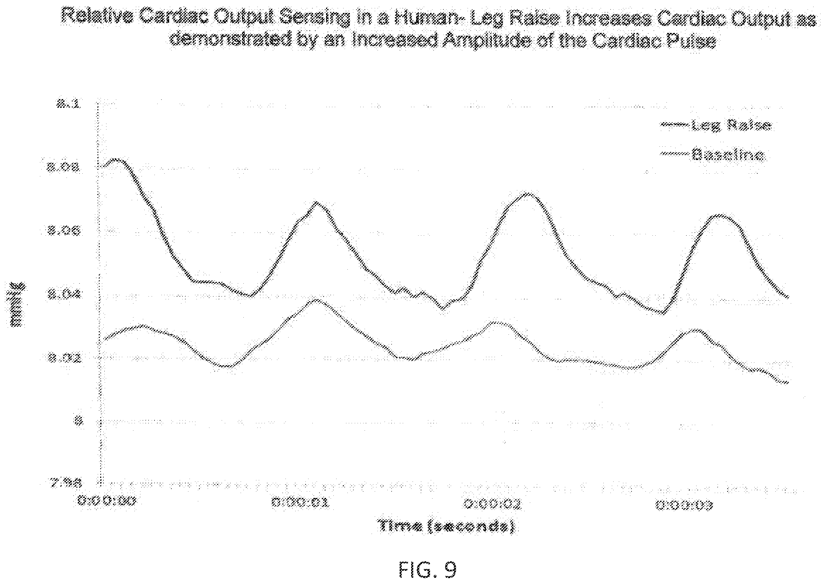

Fig. 9 shows data related to relative cardiac output sensing in a human leg raising exercise in which cardiac output increases, as demonstrated by an increased amplitude of the cardiac pulse.

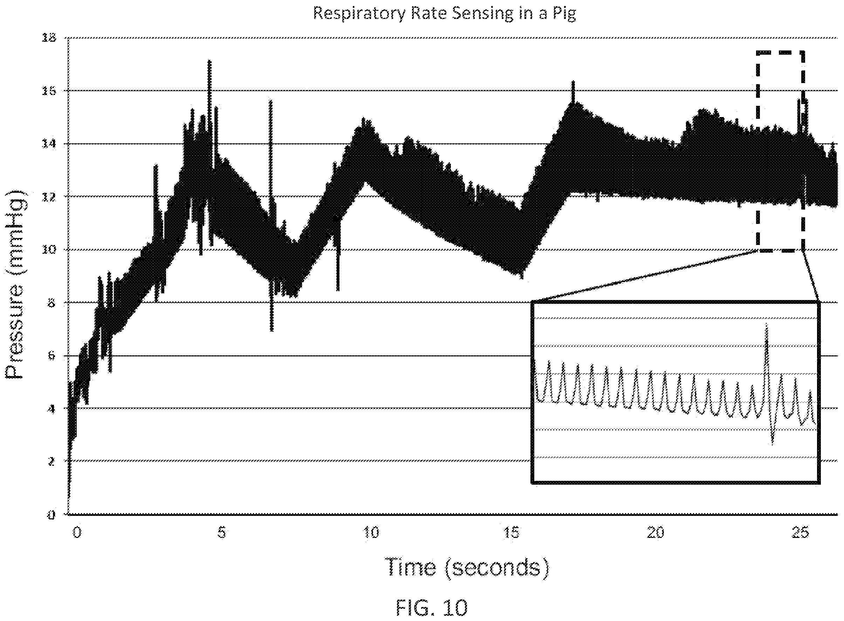

Fig. 10 shows an example of peritoneal sensing data, with a focus on respiratory rate from a pig, as provided by an embodiment of the sensing Foley catheter system.

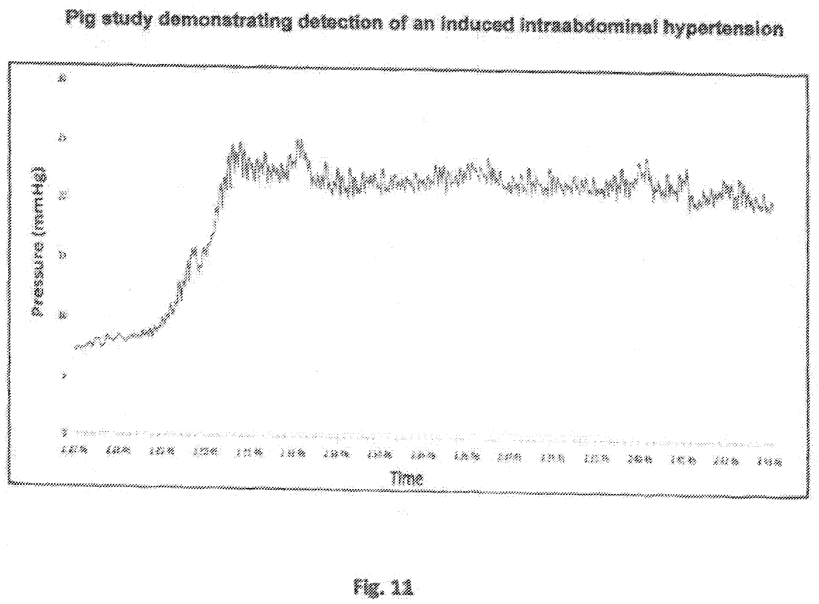

Fig. 11 shows an example of pig study that demonstrates the capability of an embodiment of the sensing Foley catheter system to detect intra-abdominal hypertension.

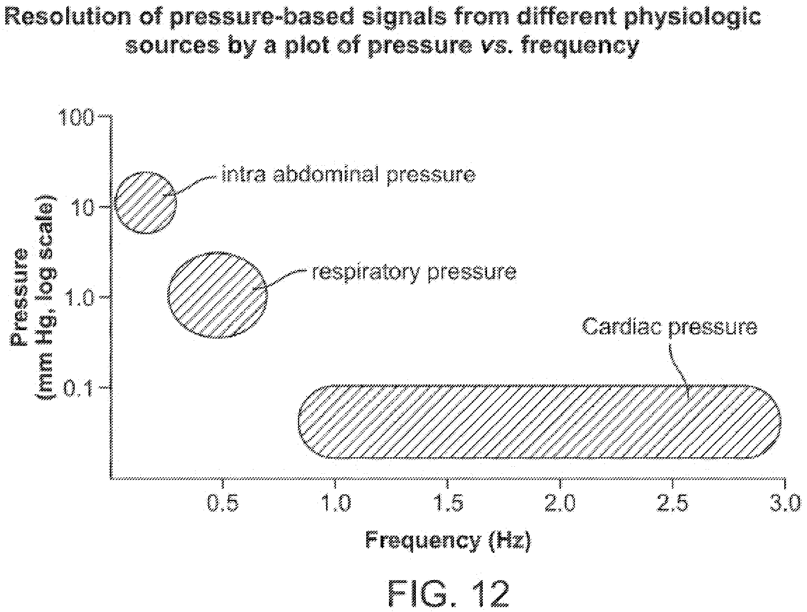

Fig. 12 shows intraabdominal pressure, respiratory wave pressure, and cardiac pressure schematically arrayed as a two dimensional plot of pressure (mm Hg on a logarithmic scale vs. frequency (Hz).

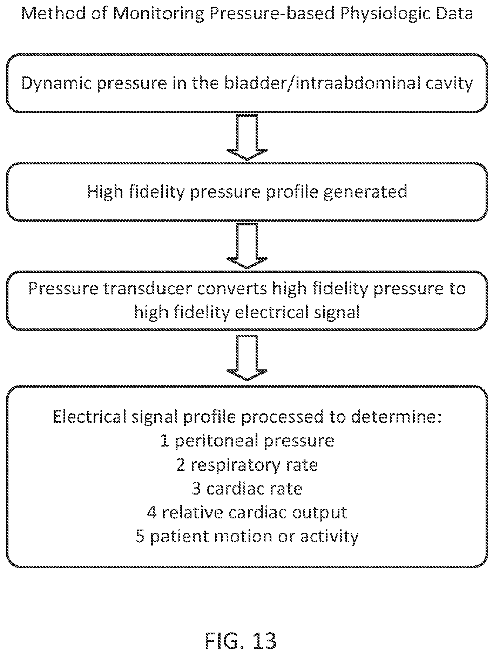

Fig. 13 provides a flow diagram of an embodiment of the method.

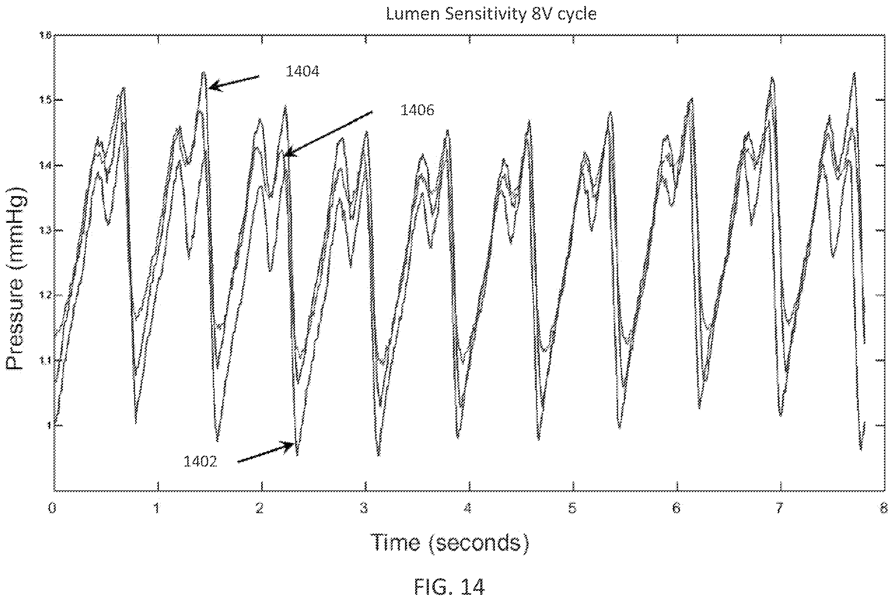

Fig. 14 shows pressure signals at different lumen diameters.

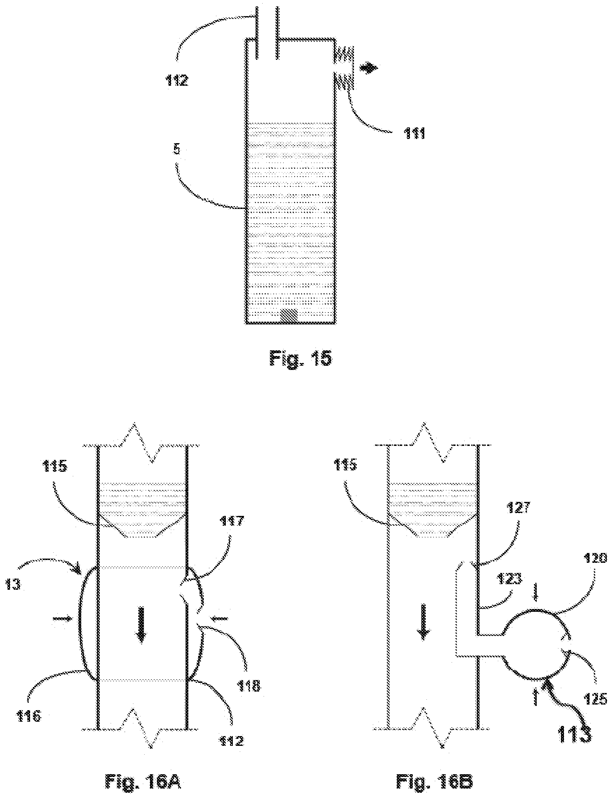

Fig. 15 shows an embodiment for clearing the drainage line that uses a vacuum applied to the end of the drainage line.
Figs. 16A-16B show an embodiment of a clearing mechanism comprising a device for positive airflow near the start of the drainage line.

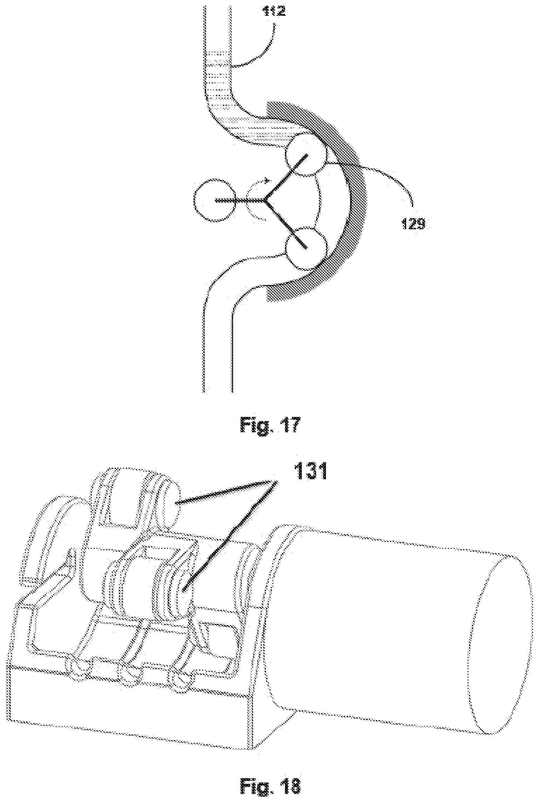

Fig. 17 shows a clearing mechanism comprising an apparatus for automated massaging, or squeezing, of the drainage line.
Fig. 18 shows another embodiment of the pinching or rolling stimulus, in which the lumens are compressed sequentially by rollers.

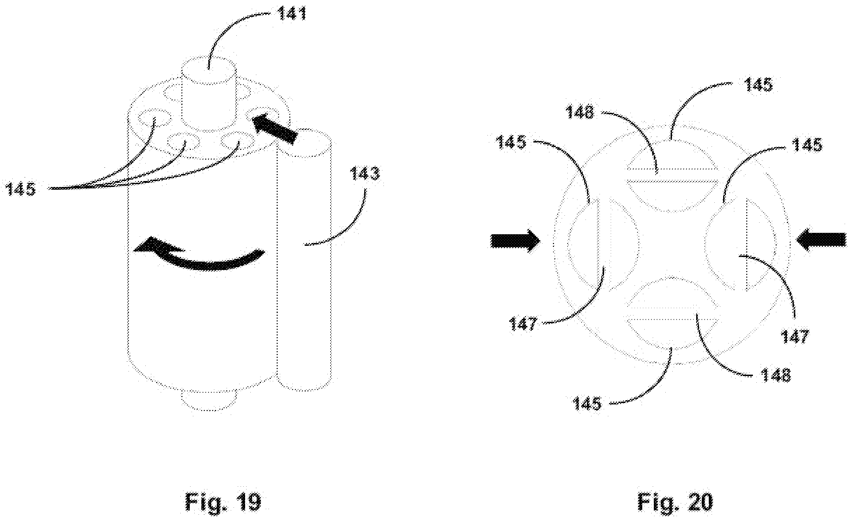

Fig. 19 shows another embodiment comprising multiple lumens organized circumferentially around a stiff member that the pinching or rolling mechanism rotates around.
Fig. 20 shows an alternative embodiment in which the lumens are organized such that they can only be completely compressed when pinched in a certain direction.

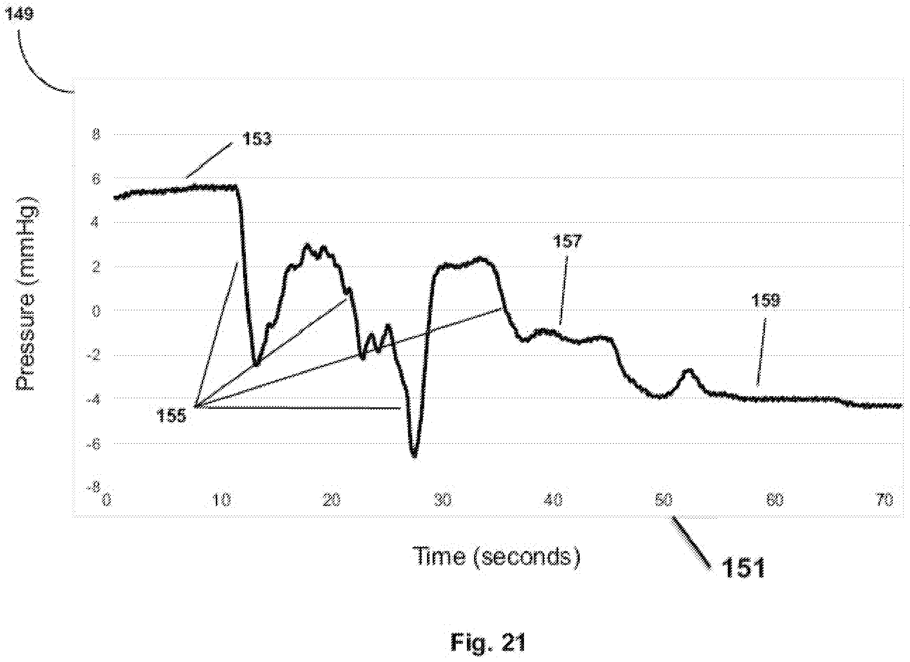

Fig. 21 shows a graph of the pressure profile, pressure (mmHg) over time (seconds) in the drain tube while the peristaltic roller pump is activated.

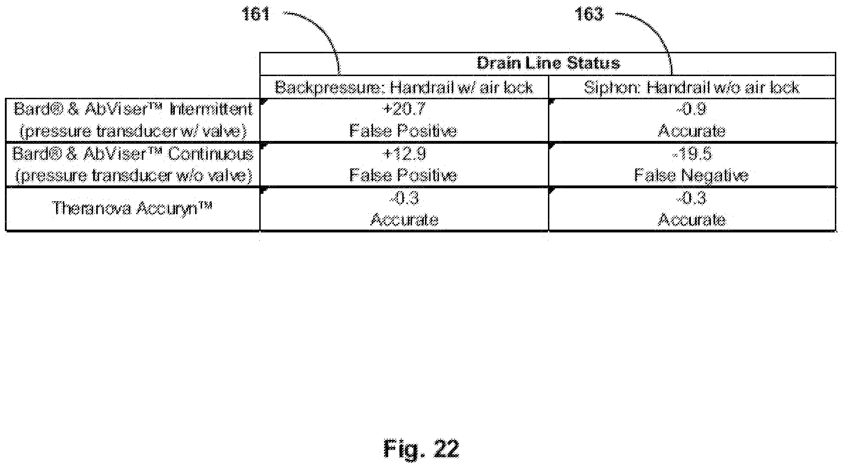

Fig. 22 is a table comparing IAP measurements using a standard drainage line and IAP sensor with the present invention in combination with a pressure-sensing Foley catheter under air lock and siphon effects.

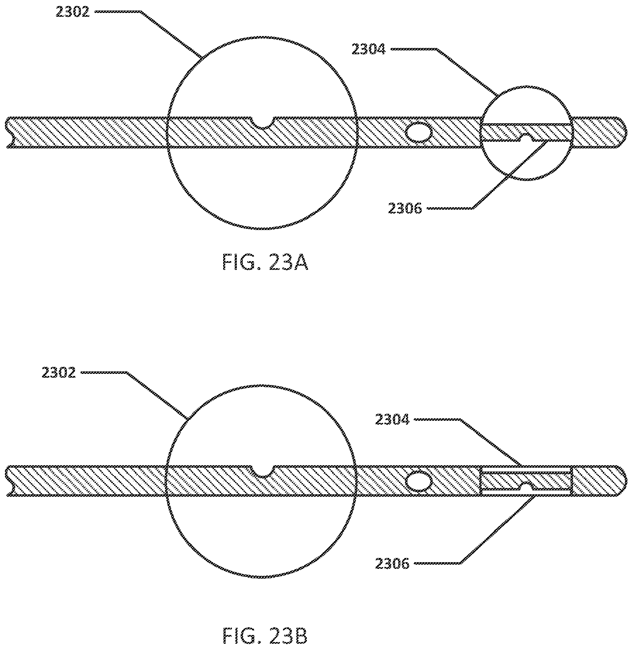

Figs. 23A and 23B show another embodiment of the disclosed technology which allows for a smaller profile catheter, particularly in the area of the pressure balloon.

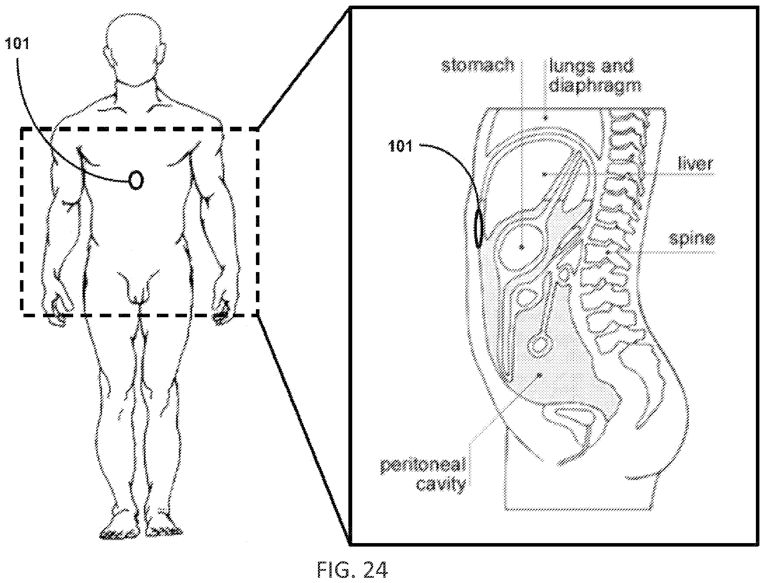

Fig. 24 shows an embodiment of a preperitoneal sensing implant.

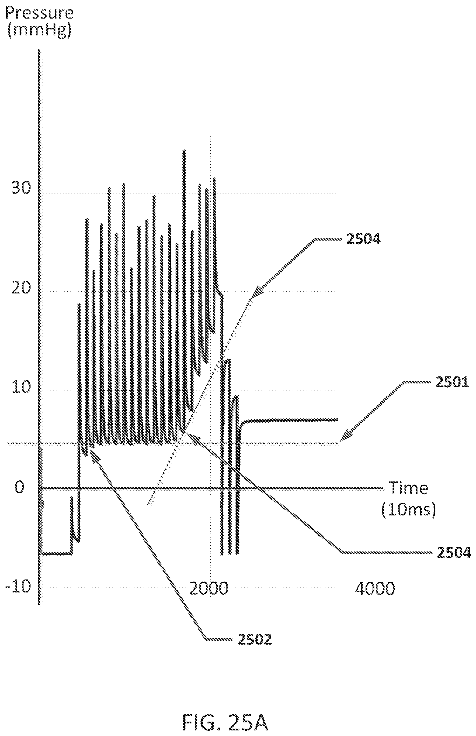

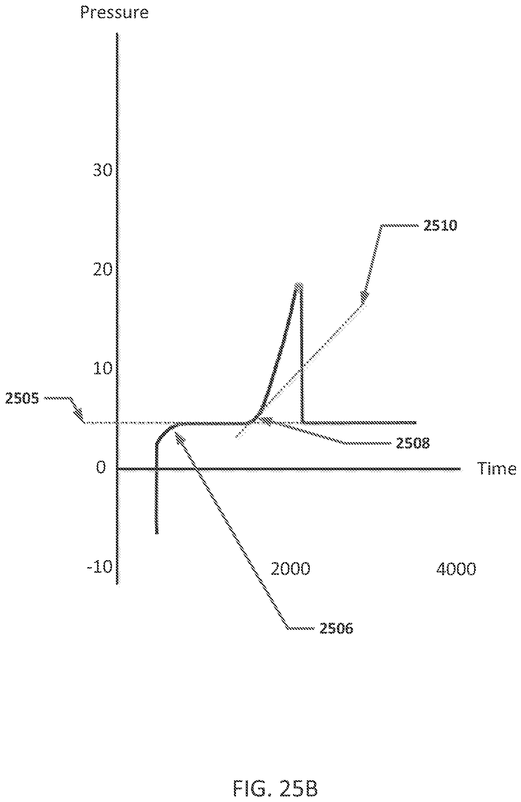

Figs. 25A and 25B show graphs representing pressure balloon priming methods in some embodiments.

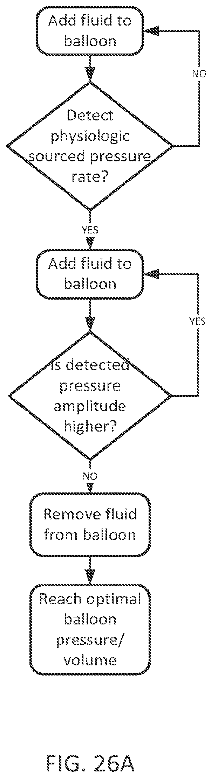

Fig. 26A-C show flow charts of possible logic in various embodiments of the invention.

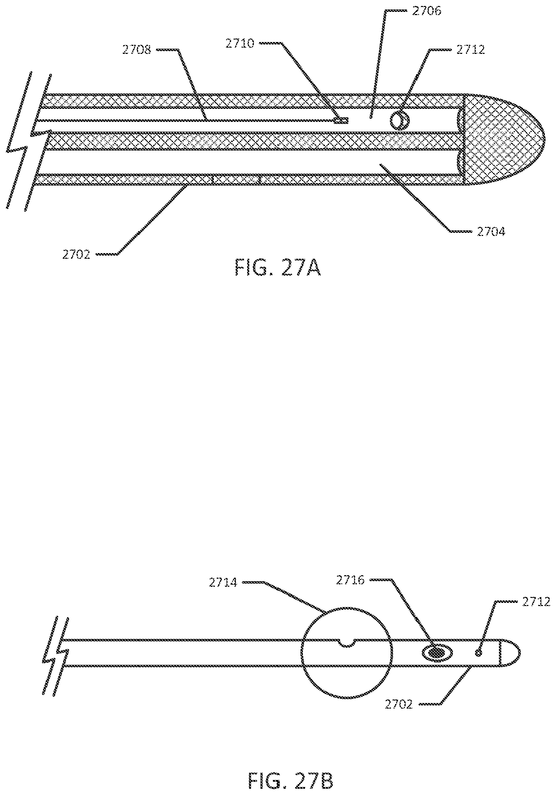

Figs. 27A and 27B show an embodiment of the invention which includes a fiber-optic pressure sensor.

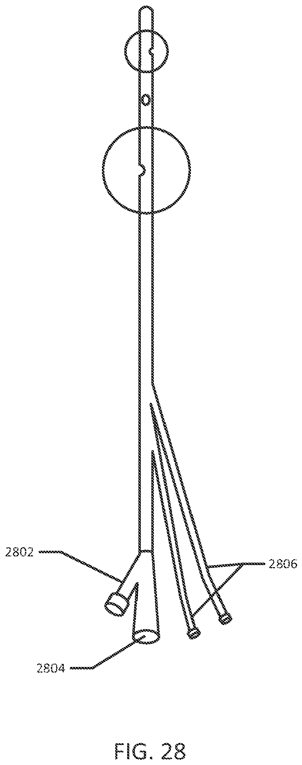

Fig. 28 shows an embodiment of the invention with more than one pressure lumen.

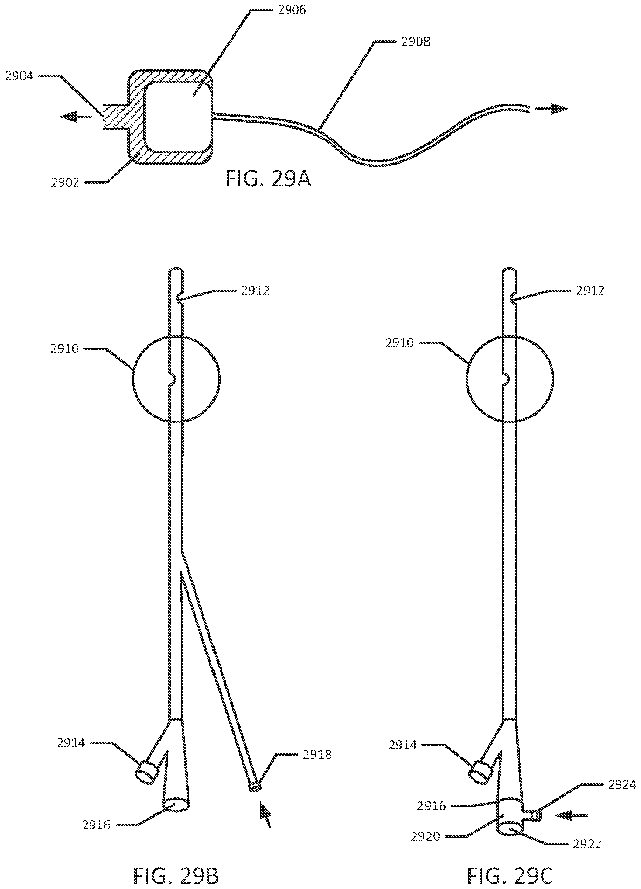

Fig. 29 shows another embodiment of the invention.

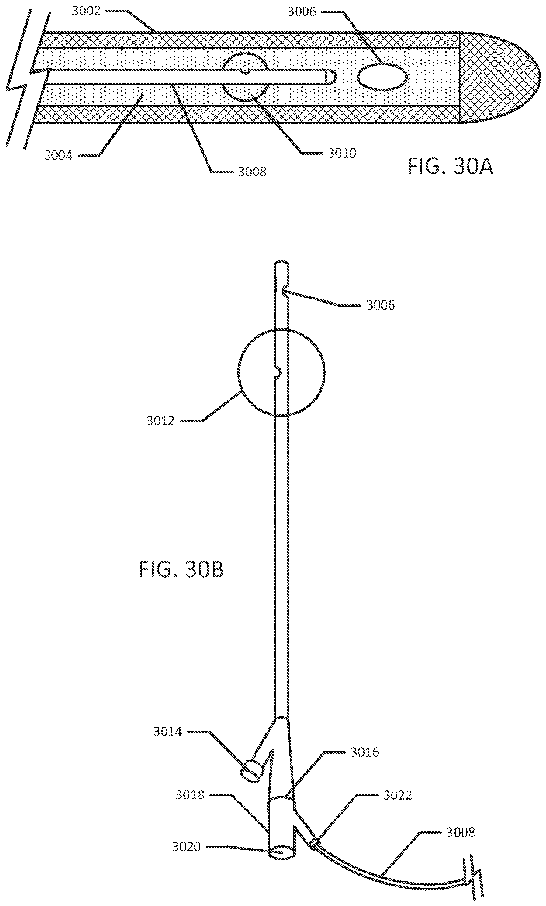

Fig. 30 shows another embodiment of the invention.

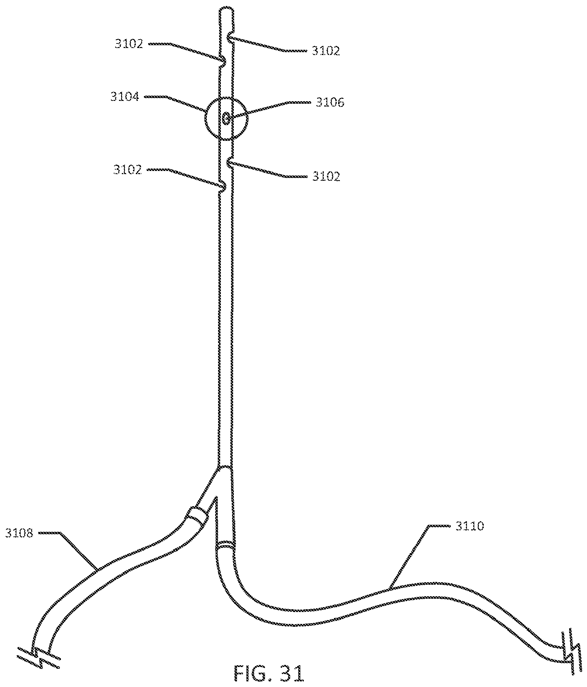

Fig. 31 shows an embodiment of the invention without a retention balloon.

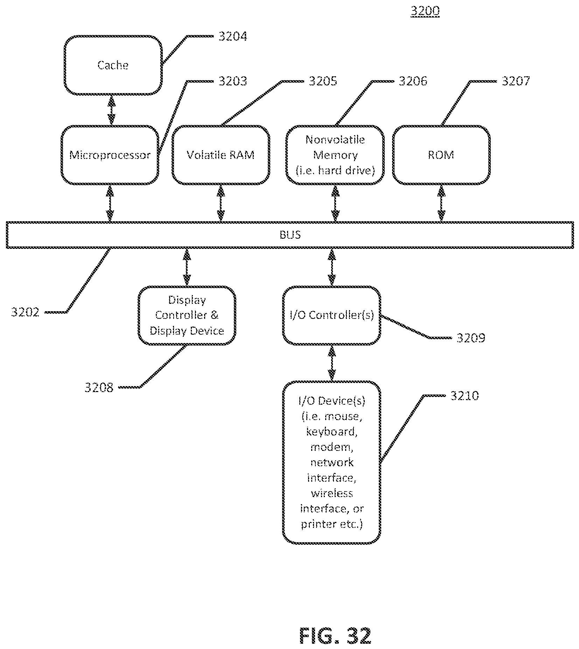

Fig. 32 is a block diagram of a data processing system, which may be used with any embodiments of the invention.

### DETAILED DESCRIPTION OF THE DISCLOSURE

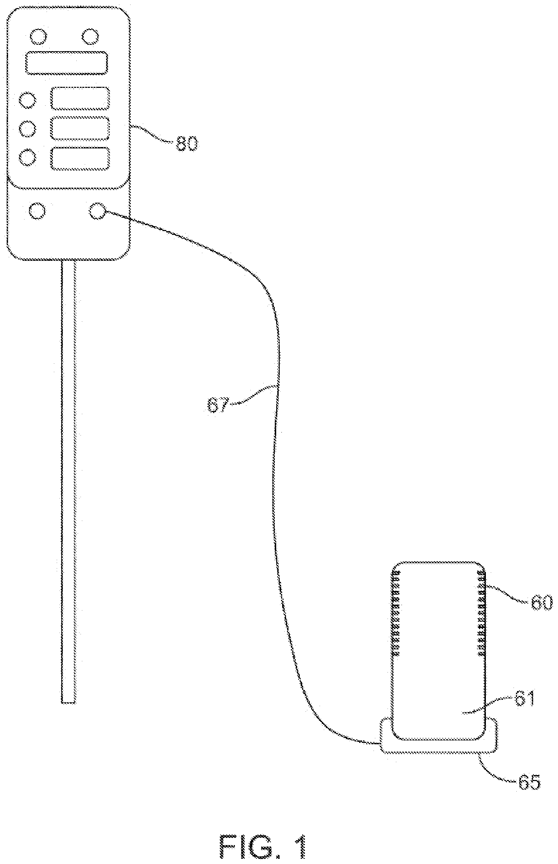

Figs. 1 - 4 show various elements of the disclosed technology, including a urine receptacle 60 (holding a urine output 61), a docking station 65 to hold the receptacle, an electrical connection 67 that allows communication between the docking station and a data collection and processing apparatus in the form a bedside console 80. Embodiments of the urine collecting receptacle 60 may include level or volume sensors 62, as well as other analyte sensors. Receptacle 60 may also include an RFID element that provides a unique identifier to a remote RFID reader 68. In some embodiments, an extender tube 63 may be utilized to convey urine from the catheter to the urine-collecting receptacle.

Fig. 1 shows a data receiving and processing apparatus in the form of a bedside console 80 in communication with a receptacle docking station 65 that accommodates a urine collecting receptacle 60, shown as holding a urine output 61, per an embodiment of the sensing Foley catheter system. The communication path between the docking station and the console may include a wired connection 67, as shown, or it may be a wireless connection. The bedside console may record and display output/input data. Physiologic data from sensors associated with a sensing Foley catheter may be held in a memory, displayed, printed, or directly transmitted to a centralized data collection server.

In some embodiments, the bedside console or controller is portable and able to travel with the patient. Embodiments of console may be attachable to a patient's bed or an IV pole, or a wall mount; it typically has its own display, and is able to provide critical alerts. Some embodiments of console may be adapted to be able to operate on a battery backup for 4 or more hours, as for example when wall power is unavailable or has been lost. This portability feature of console is advantageous in situations where patients are typically not being electronically monitored, such as when a patient is in transit from his or her bed to another location. Embodiments of console may also be configured to communicate to a base station with alerts and centralized reporting and data collection. A controller or base station may also generate mobile alerts that may be sent to nurses or healthcare provider. Signal analysis and/or predictive algorithms may also be used to provide useful clinical data from sensors.

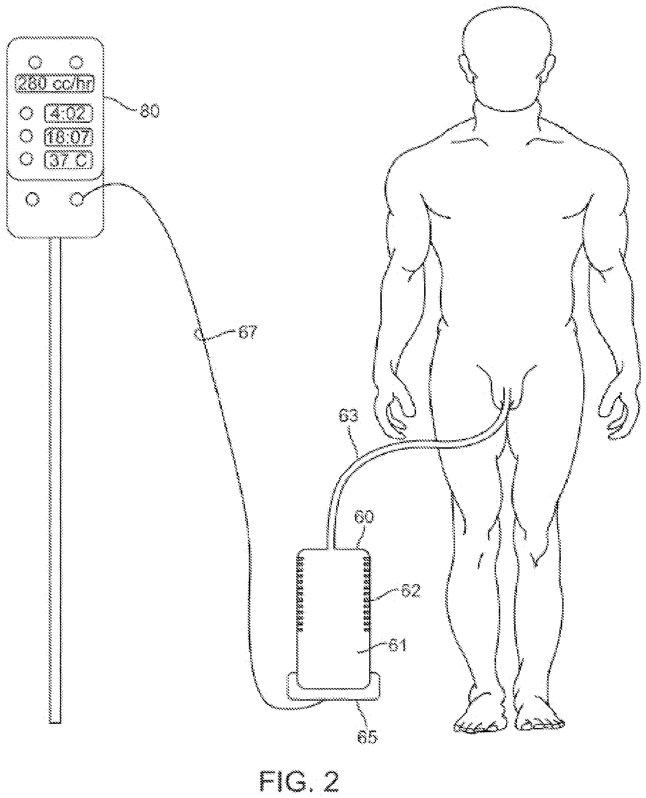

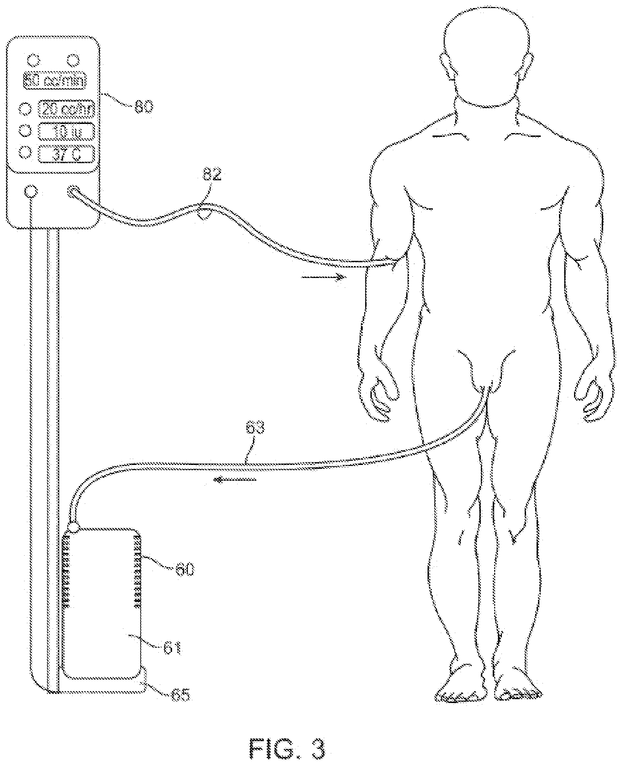

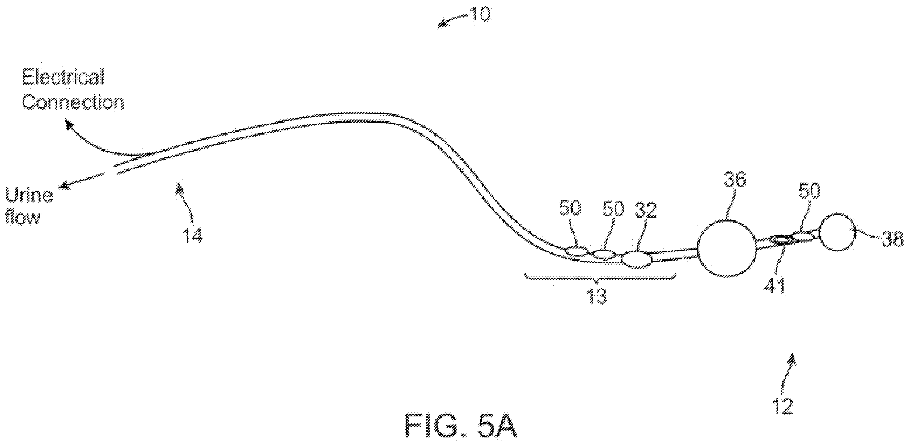

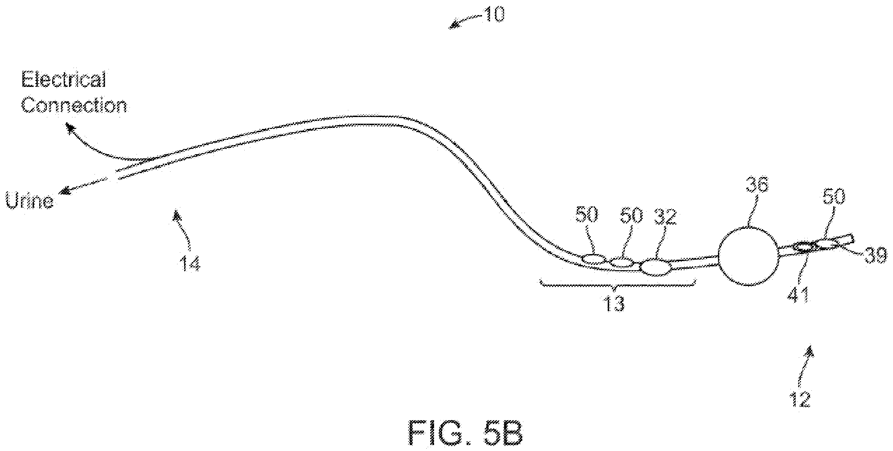

Fig. 2 shows elements of an embodiment of the sensing Foley catheter system configured to measure urine output from a human subject. In some embodiments, the bedside console 80 or an RFID reader (see Fig. 5) is enabled to trigger an alert if urine output is above or below a preset normal or desired range for urine output over a set period of time. Some embodiments of the system may also have an intravenous infusion capability (see Fig. 3) to provide use sensed data to regulate delivery of fluids or medicinal agents such as a diuretic drug, by way of an automated system based on the urine output feedback. Embodiments of the system may include a docking station for the urine collecting receptacle, the docking station being configured for data transmission to a data receiving and processing apparatus such as a bedside console or a networked central computer. In some embodiments, the docking station delivers data regarding the volume of urine in the urine receptacle, as well as data that are informative regarding electrical parameters of the urine, such as conductivity, resistance, or impedance. Sensors may also detect and monitor bacteria, hemoglobin, or other substances of clinical significance in urine. Sensors may also measure urine opacity in the collecting receptacle, in the bladder or in the catheter/tubing.

Fig. 3 shows an embodiment of the sensing Foley catheter system configured as an automated infusion therapy system for a human subject. A bedside console 80 may integrate patient data, such as fluids received or urine output recorded, and then automate therapeutic infusion in response to these data. For example, delivery of fluids or drug solutions such as a physiological saline solution may be initiated or regulated through an infusion line 82 if the patient is dehydrated, or a diuretic may be infused if the patient is fluid overloaded. In some embodiments, the console may trigger a local alert (e.g., audible beeping), or trigger a centralized alert (e.g., a system alarm) if urine output drops too low. The console may also integrate a hydrating or medicinal fluid infusion capability, such as an IV infusion pump, and may adjust infusion rates based on these data or based on data acquired from other sensors automatically. The console may communicate wirelessly, as well, to these and other sensors within the body.

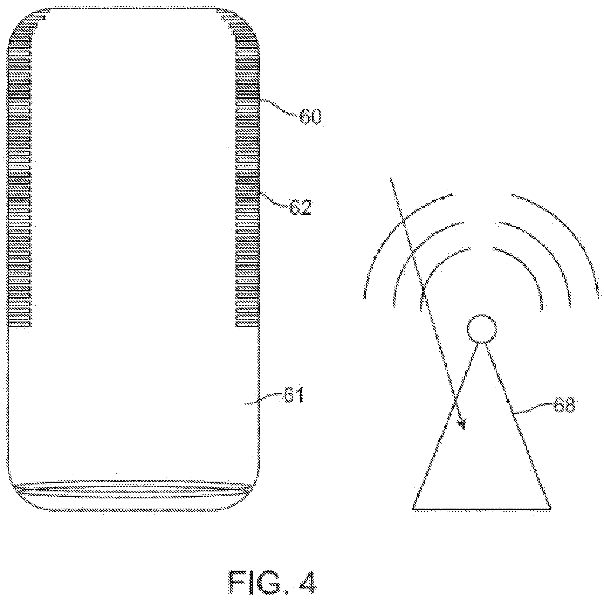

Fig. 4 shows a volume-sensing urine receptacle 60 accommodated within a receptacle docking station 65, per an embodiment of the sensing Foley catheter system. Embodiments of the receptacle may detect urine output based on the levels at which sensors 62 are triggered. For example, the receptacle may electrical contacts arranged as liquid height-marks, and when an electrical path is made between two contacts and all contacts below, the level can be reported at that level. Embodiments of the receptacle may include electrical, optical, chemical or mechanical sensors. Embodiments of the receptacle may include also contain diffuse or discrete sensing areas that detect analytes of interest, e.g., hemoglobin, protein, glucose, bacteria, blood, leukocyte esterase. Sensing or data reporting of sensed data may be of either an intermittent or a continuous nature.

Embodiments of the receptacle may include a capability to report sensing data to the bedside console, locally (e.g., by beeping) or centrally via piping data to a central information collection area. For example, an alert may be triggered if urine output drops below 30 cc/hr. in post-operative setting or below any otherwise predetermined threshold. Embodiments of the receptacle may connect to a docking station through electrical contacts; data communication among embodiments of the receptacle, docking station, and a console or central computer may also be wireless. If a docking station is used, it may detect urine output based on weight or pressure of the receptacle that is applied to base.

Embodiments of the urine collecting receptacle may include disposable or durable optical, electrical or chemical sensors capable of sensing and measuring urine content of analytes such as glucose, electrolytes, bacteria, hemoglobin, or blood. Embodiments of the receptacle may include an interface with specifically designed area of the urine receptacle to allow for this measurement, such as an optically clear window for optical measurement of blood. Embodiments of the receptacle docking station may also grasp or accommodate the urine receptacle in any manner so long as it secures the receptacle. The docking station or the receptacle may include an inductive antenna or RFID capabilities to allow for wireless querying and reporting of the level of urine or other fluid collection.

The embodiment of Fig. 4 also shows a volume-sensing urine receptacle 60 that includes an RFID chip, per an embodiment of the sensing Foley catheter system. This embodiment may contain RFID circuitry to collect and transmit data directly from within the receptacle to a remote RFID reader 68. When queried by the RFID reader, the receptacle may detect impedance, resistance, capacitance or any other electrical or non-electrical property to measure the urine level and report this back to the reader. The reader may then trigger alert if urine output is out of a normal or desirable range. The RFID chip may be capable of detecting changes in optical, chemical, electrical, acoustic or mechanical properties, as well. RFID chips may be active or passive, and may contain an antenna to transmit a receptacle-identifying signal to the reader, and allow multiple receptacles to be queried simultaneously. An active RFID chip may incorporate a small battery (to extend its range). A passive RFID chip may be powered by the transmission from the RFID reader. The RFID reader may query a device from a distance to wirelessly check the urine output level or it may be centralized to query all receptacles within a unit, floor or hospital and issue an alert if urine output is out of a normal or desirable range. The RFID reader record urine output, as well, and functionally replace the individual unit console shown in Figs. 1 - 3. The RFID reader may also report data from other sensors within the system, including bladder temperature or presence of analytes (as detailed elsewhere) in the urine.

Figs. 5A - 6D show embodiments of a sensing Foley catheter 10 and various of its features. A catheter may be understood to have various sections according to their disposition when the catheter is inserted into a human subject, such as a proximal portion 14 that remains external to the subject, a central or urethra-residing portion 13, and a distal or urinary bladder-residing portion 12.

Various internal lumens traverse the length of the catheter, such as an air or fluid 24 that communicates with a bladder retention balloon 36. A urine drainage lumen 23 has a distal opening 41 that resides in the bladder portion 12 of the catheter, and has an opening at the proximal end 14 of the catheter. As seen in Figs. 2 and 3, the urine drainage lumen may be connected to an extender tube 63 that conveys the urine to a collecting receptacle. In some embodiments, the drainage lumen and distal opening in the bladder may also serve as in infusion conduit (see Fig. 3) by which medicinal agents may be infused, or through which heating or cooling fluid may be infused. Analyte sensors or temperature sensors 50 may be disposed on the catheter, either on the urethral portion 10 or the bladder-residing portion 12 of the catheter. Electrical or optical fiber leads may be disposed in a lumen 25 that allows communication of sensing signals between distally disposed sensors and the proximal portion of the catheter, and then further communication to a data processing apparatus.

An inflatable pressure-sensing balloon 38 (Figs. 6A, 7A, and 7B) or a pressure sensing membrane 39 (Fig. 7B) arranged across an opening may be positioned on the distal end 12 of the catheter, residing in the bladder. Embodiments of a pressure-sensing balloon or pressure sensing membrane may be understood as comprising a pressure interface having a distal-facing surface exposed to pressure from within the bladder, and a proximal-facing surface exposed to a proximal fluid column. Embodiments of the fluid column (filled with either liquid or gas) may comprise a dedicated lumen, or such column may share a lumen that also serves as a sensing conduit such as lumen 25.

Fig. 5A shows a sensing Foley catheter that includes a pressure interface in the form of pressure-sensing balloon, per an embodiment of the presently disclosed system. Pressure-based physiologic parameters that this catheter embodiment can sense may include, by way of example, peritoneal pressure, respiratory rate, and cardiac rate, relative pulmonary tidal volume profile, cardiac output, relative cardiac output, and absolute cardiac stroke volume. Some embodiments of the Foley type catheter may be further equipped with any of a temperature sensor, one or more analyte sensors, electrodes, and paired light sources and sensors. Embodiments thus further equipped are capable of delivering other forms of physiologic data, as for example, blood pressure, oxygen saturation, pulse oximetry, EKG, and capillary fill pressure.

**Fig. 5B** shows a sensing Foley catheter with a lumen (the third lumen, for example) used as a pressure sensing lumen; this embodiment does not include a dedicated pressure-sensing balloon as does the embodiment of Fig. 5A, but instead has a pressure interface in the form of a membrane arranged over a distal opening of the pressure sensing lumen. In this embodiment, the sensing Foley catheter is able to detect and report pressure-based physiologic data as included in the embodiment described above. In this present embodiment, a slow infusion of fluid into the bladder may be accomplished through the third lumen of a standard 3-way Foley catheter, and pressure may be sensed using a pressure sensor in line with this third lumen. In this embodiment, all methods associated with processing and responding to pressure-based physiologic data, as described for embodiments with a pressure-sensing balloon, are enabled.

Figs. 6A - 6D show various views and details of a sensing Foley catheter, per an embodiment of the sensing Foley catheter system. Fig. 6A schematically arranges the sensing Foley catheter into a proximal section 14 that remains external to the body when in use, a portion 13 that resides in the urethra, and a distal portion 12 that resides in the bladder, when placed into a human subject. Fig. 6B shows a detailed view of the proximal portion of the catheter, focusing on luminal openings 23, 24, and 25, which are configured to make more proximal connections. Fig. 6C shows a cross sectional view of the central length of the catheter, and an example of how lumens 23, 24, and 25 may be arranged. Fig. 6D shows a detailed view of the distal portion of the catheter that resides in the bladder, with a particular focus on a retention balloon 36 and a pressure-sensing balloon 38.

Pulse oximetry elements allow for a determination of blood oxygen concentration or saturation, and may be disposed anywhere along the urethral length of the catheter. In some embodiments, the sensor or sensors are disposed within the tubing of the device to ensure approximation to the urethral mucosa. With this technology, a healthcare provider can decompress the bladder with a urinary catheter and obtain pulse oximetry data in a repeatable and accurate manner. The power source for pulse oximetry may be incorporated within the urinary collecting receptacle or within the catheter itself. In some embodiments, the pulse oximeter is reusable and the catheter interface is disposable; in this arrangement the pulse oximeter is reversibly attached to the disposable catheter and removed when oxygen measurements are no longer desired. Embodiments of the sensing Foley catheter may include an optically transparent, or sufficiently transparent, channel for the oximetry signal, such as a fiber-optic cable, transparent window, and an interface for the reusable oximeter. This method and device for urethral pulse oximetry may be used in conjunction with any of the other embodiments detailed herein or may be a stand-alone device.

Embodiments of the sensing Foley catheter may be able to sense any one or more of a plurality of clinically relevant parameters, such as included in the following examples: urine pH, urine oxygen content, urine nitrate content, respiratory rate, heart rate, perfusion pressure of the bladder wall or the urethral wall, temperature inside the bladder or the urethra, electro-cardiography via sensors on the bladder wall or the urethra, respiratory volume, respiratory pressure, peritoneal pressure, urine glucose, blood glucose via urethral mucosa and/or bladder mucosa, urine proteins, urine hemoglobin, blood pressure. In some embodiments, the catheter can sense multiple parameters, but some embodiments may be limited to as few as a single parameter for focused applications (for example, respiratory rate in a patient in respiratory distress). The respiratory rate, relative tidal volume, peritoneal pressure, heart rate and/or relative cardiac output may be measured simultaneously, as well, by connecting a balloon with a flaccid wall or semi-tense wall to an external pressure sensor via a lumen that may be filled with liquid and/or gas.

These parameters may be measured, alone or in concert with other parameters, through the use of pressure measurement modalities other than the external pressure sensor. These may include: a deflecting membrane inside of the catheter, MEMs technology, a catheter-based sensor and/or other embodiments.

Relative cardiac output and relative tidal volume may also be calculated, based on the deflection of the pressure sensor and/or other force gauge. If sampled with sufficient frequency (e.g., 1 Hz or greater), respiratory excursions can be quantified in a relative manner to the amplitude of the excursions at the time of catheter placement. Larger excursions generally relate to heavier breathing, or in the setting of an upward drift in the baseline, a higher peritoneal pressure. The small peaks on the oscillating respiratory wave, caused by the pumping heart, may be tracked as well by using faster sampling rates (e.g., 5 Hz or greater), and the amplitude of this wave may be used, in the setting of a relatively constant peritoneal pressure, to determine the relative cardiac output, in the setting of a known, stable peritoneal pressure, absolute stroke volume and/or cardiac output.

The disclosed technology captures a high-resolution chronological profile (pressure as a function of time) of peritoneal pressure that can be transduced and processed into distinct pressure profiles assignable to particular physiologic sources, including peritoneal pressure, respiratory rate, and cardiac rate. By tracking the pressure profile at a sufficiently rapid sampling rate, as provided by the technology, the pressure profile can be further resolved into relative pulmonary tidal volume, cardiac output, relative cardiac output, and absolute cardiac stroke volume.

Accordingly, aspects of the disclosed technology relate to fidelity and resolution of a pressure signal generated in response to changes in pressure within the bladder, such changes being reflective of a pressure profile within the peritoneal cavity, such pressure profile including cumulative input from the aforementioned physiologic sources. Aspects of the technology further relate to fidelity and resolution of the transduction of the pressure signal into a highly resolvable electrical signal. Aspects of the technology relate still further to processing the totality of the electrical signal profile, a surrogate for the pressure profile within the peritoneal cavity, into component profiles that can be assigned to the physiologic sources.

The sensitivity of an inflated balloon as a pressure sensor is a function, in part, of the pressure differential across the balloon membrane as a baseline condition. The balloon has the greatest sensitivity to pressure when the baseline pressure differential is near zero. As the baseline pressure differential increases, the sensitivity of the pressure-sensing balloon degrades. Accordingly, the disclosed technology provides an automatic priming method that maintains the balloon in an inflated state, but with a minimal pressure differential.

Embodiments of the technology include a pressure interface as may be represented by a balloon having either a compliant membrane or a non-compliant membrane. In general, considerations related to optimizing the pressure around the pressure interface of the device are informed by Boyle's ideal gas law, the relationship between stress and strain as described by Hooke, and by application of Young's modulus. The conditions for optimal sensitivity of a compliant balloon and a non-compliant balloon are slightly different, although, in general, the sensitivity of each is best served by P1 and P2 being approximately equal. A non-compliant balloon maximum sensitivity is achieved when P1 is only slightly above P2. For a compliant balloon, the maximum sensitivity is achieved when P1 is slightly above P2 at the low end of the (linear) elastic region of the spring constant of the compliant balloon material.

To effectively capture physiologic pressure profiles, the profiles need to be sampled at a rate that is sufficient to resolve the inherent frequency of changes in the profile. This consideration is informed by the Nyquist-Shannon sampling theorem, which states that a sampling frequency of at least 2B samples/second is required to resolve an event that runs at a frequency of B cycles/second. As applied to a physiologic pressure cycle, for example, a cardiac rate of 70 beats/minute requires a sampling rate of at least 140 samples/minute to effectively capture the cycle. This relationship underlies aspects of the disclosed technology that specify the sampling rate particularly required to capture physiologic pressure cycles such as relative pulmonary tidal volume, cardiac output, relative cardiac output, and absolute cardiac stroke volume.

Fig. 12 shows intraabdominal pressure, respiratory wave pressure, and cardiac pressure schematically arrayed as a two dimensional plot of pressure (mm Hg on a logarithmic scale vs. frequency (Hz). It can be seen that there is an inverse relationship between pressure and frequency, and the various physiologic pressure-related parameters occupy distinct sectors when arrayed in this manner. It is by the distinctness of these profiles that embodiments of the method (see Fig. 14), as disclosed herein, can resolve a single overall chronological pressure profile into the distinct subprofiles, in accordance with their physiologic origin.

Expandable pressure sensing balloons, per embodiments of the technology, may assume one of at least two basic forms, type 1 or type 2. In balloon embodiments of type 1, which may be generally likened to a conventional party balloon, the pressure-sensing balloon is formed from or includes a compliant or elastic membrane. Accordingly, the surface area of the membrane expands or contracts as a function of the expansion of the balloon. The elasticity of the membrane determines various features of the balloon, as a whole, at different levels of expansion. Upon expansion, the balloon, if unconstrained, maintains a substantially constant or preferred form or shape, as determined by the mandrel upon which the balloon is formed. Upon expansion of the balloon from a minimal volume to its maximal volume, the membrane of the balloon maintains a level of tautness. Within the limits of elasticity of the compliant membrane, an increase in pressure during inflation results in a consequent expansion of volume. The balloon, on the whole may be considered partially compliant in that its shape responds to spatial constraints that it may encounter upon expansion or inflation, however the balloon does have a preferred or native shape, and such shape preference prevents a level of shape compliance or conformability such as that shown by a balloon of type 2.

In balloon embodiments of type 2, the expandable pressure-sensing balloon is formed from or includes a non-compliant, or non-elastic membrane, or a membrane that is substantially non-compliant or non-elastic. Accordingly, the surface area of the membrane does not expand or contract in accordance with the level of balloon expansion. Type 2 pressure-sensing balloons may be generally likened to a conventional Mylar® balloon. The inelasticity of the membrane determines various features of the balloon, as a whole, at different levels of expansion. Upon expansion of the balloon from a minimal volume to a level near its maximal volume, the membrane of the balloon is supple, and has a level of slackness. Expansion of a type 2 balloon occurs by way of outwardly directed smoothing of wrinkles and folds in the membrane. Deflation or compression of a type 2 balloon occurs by way of generally inwardly directed wrinkling and infolding. When a type 2 balloon is fully inflated (or substantially inflated) without being in a confining space, it assumes a preferred or native shape as determined by the geometry of the membrane or fabric of the balloon. However, in a state of partial inflation, the balloon, as a whole, is highly supple and conformable, broadly taking the shape as may be dictated by a confining space.

Expandable pressure sensing balloons, per embodiments of the technology, may also include features of both of the two basic forms, type 1 or type 2. In these embodiments, the membrane may include regions that are elastic (like type 1) and regions that are inelastic (like type 2). A balloon of this hybrid type would, as a whole, behave in a manner drawing from behavioral aspects of both type 1 and type 2 balloons, as described above. Further, type 1 balloons may be formed with a membrane that is not of a homogeneous composition or thickness. In such embodiments, regions of different thickness or composition could have varying degrees of elasticity, thus affecting the behavior of these regions during expansion of the balloon. In still other embodiments, elasticity of the membrane may have a bias or polarity that tends to permit elasticity in one or more directions, and tends to disallow elasticity in one or more other directions.

An aspect of the disclosed technology that is particularly advantageous in achieving a high resolution signal from which pressure profiles from particular physiologic sources (such as peritoneal pressure, respiratory rate, and cardiac rate, relative pulmonary tidal volume, cardiac output, relative cardiac output, and absolute cardiac stroke volume) may be monitored relates to adjusting and maintaining a balance of pressure on either side of the pressure interface represented by the membrane of the pressure sensing balloon. This balance of pressure may be referred to as a pressure differential of zero, or as a zero pressure gauge. Pressure impinging on the external face of balloon (facing the internal aspect of the bladder) is subject to change according to the physiology of the patient. Pressure on the internal face of the balloon (which is in fluid communication with the fluid column) is subject to degradation because of fluid leakage and imperfect seals.

Upon first insertion of the Foley type catheter, external pressure is typically applied to the fluid column and against the pressure interface to a first approximation of pressure being exerted on the pressure interface from within the bladder. Pressure signals, as measured across a pressure interface, have a maximal amplitude when the pressure differential is zero. Accordingly, the amplitude of a pressure signal can be used to tune the pressure being applied from the fluid column against the pressure interface. This process of applying an appropriate amount of pressure against the interface may be referred to as priming the fluid column or priming the balloon. Inasmuch as pressures on either side of the pressure interface may change, as described above, the fluid column may need to be reprimed or re-tuned, from time to time. The necessity of repriming can be monitored by testing small changes in pressure so as to achieve maximal amplitude of a pressure signal profile.

Embodiments of the disclosed system and method include automatic pressure tuning by a controller. Accordingly, the tuning system can detect the optimum target pressure and volume to inflate the balloon by monitoring sensed pressure signals and adding or removing air or fluid volume as needed. For example, upon insertion of the catheter, a pressure tuning circuit that regulates the balloon volume and pressure may inflate the balloon until it detects a physiologic-sourced pressure rate. Upon sensing that rate, the pressure tuning controller may add or subtract minute amounts of air in a routinized sequence until the amplitude of the sensed wave is greatest The control feedback loop between the optimally tuned pressure (manifesting as balloon pressure and volume) and the sensed physiologic pressure profile iterates continuously and or as needed to ensure high fidelity measurement of the physiologic data. In some embodiments, automatic pressure tuning may be performed in the apparent background while the physiologic data is being transmitted and displayed; in other embodiments the system may suspend transmission of physiologic data during a pressure tuning sequence.

Embodiments of the disclosed technology include a gas delivery system that can deliver gas in a priming operation, whereby pressure can be applied to a fluid column proximal to the proximal-facing aspect of the pressure interface. A source of gas, such as compressed air or liquid is held in a storage tank. Using CO₂ as an example, CO₂ is controllably released from the storage tank through a pressure regulator that can step pressure in the tank (for example, pressure of about 850 psi) down to the range of about 1 psi to about 2 psi. Released gas passes through a filter and a pressure relief valve set at about 2.5 psi. The pressure relief valve is a safety feature that prevents flow through of gas at a level greater than 2.5 psi in the event of failure of the upstream regulator. CO₂ exiting the pressure relief valve next passes through a first solenoid-controlled fill valve to enter the catheter line, ultimately filling the balloon that comprises the pressure-sensing interface. Pressure within the balloon is allowed to rise to a level as high as 30mm Hg, whereupon the first solenoid-controlled valve closes. A second solenoid-controlled valve, distal to the first valve operates as a drain valve, which can release pressure from the catheter to a target pressure. Alternatively, the drain valve may be activated until a respiratory waveform is detected after which the balloon will be optimally primed and the valve will be closed. The drain valve may be subject to proportional control, operably based on voltage or pulse-width modulation (PWM), which allows a drain rate sufficiently slow that the target pressure is reached and the valve can be closed prior to overshoot Alternatively, a peristaltic or other air pump may be utilized to fill the balloon with room air.

Intrabdominal pressure or bladder pressure, as sensed by an embodiment of the disclosed technology, may also be used to detect the level of patient movement (as may vary, for example, between substantially no movement to a high level of movement) and to report the movement level to a healthcare provider. A short burst of peaks and valleys in bladder pressure activity can serve as a proxy for body movement in that such a bladder pressure profile is a strong indicator that the patient is using their abdominal muscles, as, for example, to sit up or get out of bed. This embodiment may be of particular benefit for patients that are at risk of falling. In a patient that is a fall-risk, a healthcare provider may be notified that the patient is sitting up and respond accordingly. Alternatively, the device may be used to report inactivity of a patient and/or lack of patient movement.

Embodiments of the technology may also report patient movement in the detection or diagnosis of seizure disorder. In this embodiment, the pressure variations may trigger an EEG or recording equipment to allow for intense period of monitoring during an episode suspected of being a seizure. In addition, or alternatively, a pressure sensor, acoustic sensor or other sensors may be used to detect bowel activity, including peristalsis, patient movement, seizure activity, patient shivering, frequency of coughing, severity of coughing, sleep duration, sleep quality, speech detection, patient compliance (movement or lack thereof), and may alert the healthcare provider that the patient has not moved and must be turned or rolled. This movement-related information may also be relayed to a hypothermia device, a drug delivery device or other device to control or mitigate seizure activity, shivering and/or coughing.

Embodiments of the technology may also automatically adjust intravenous fluid or drug infusion rates based on feedback from the cardiac output or respiratory rate sensed. In one such embodiment, a patient-controlled analgesia pump may be deactivated if a respiratory rate drops too low. Respiratory depression can be fatal in this group and this safeguard would prevent overdose. An automated feedback system may also be advantageous in a large volume resuscitation procedure, wherein fluid infusion can be tailored based on intraabdominal pressure to prevent abdominal compartment syndrome by sounding an alert and slowing infusion rates as the intraabdominal pressure rises. Yet another automated feedback feature may provide direct feedback to a ventilator system to provide the optimal pressure of ventilated gas. In the setting of increased abdominal pressure, typical ventilator settings do not provide sufficient respiration for the patient. An automated adjustment of the ventilator settings based on intraabdominal pressure feedback from this embodiment may advantageously provide for optimal patient ventilation. Embodiments of the technology may also be applied as a correction in the application or understanding of other diagnostic measurements. For example, central venous pressure may be dramatically distorted in the setting of elevated intraabdominal pressure. Providing direct access to these data by the central venous pressure reporting system allows for the automatic correction and accurate reporting of this critical physiologic parameter. Embodiments of the technology may also be used in a variety of other ways to automate therapy including infusion of fluids that may further include active agents, such as pressors or diuretics in response to increased or decreased cardiac output.

In some embodiments, the Foley type catheter is configured to report the presence of a water droplet or other obstruction in an air-filled lumen, and then handle or resolve the droplet. In a hypothermic setting, in particular, moisture in an air lumen can condense and form obstructive water droplets. Water droplets in an air-filled lumen (or air bubbles in a water-filled lumen) can disturb or complicate pressure signals due to the surface tension of the water. Accordingly, a pressure-transmission lumen in some embodiments of the disclosed technology may include a hydrophilic feature (such as a coating on the wall of the lumen itself, or a hydrophilic fiber running the length of the lumen) to wick moisture away from the lumen in order to maintain a continuous, uninterrupted air channel. In some embodiments, a hygroscopic composition (silica gel, for example) may be used in line with the air infusion line or within the air infusion lumen itself to capture water or humidity. In some embodiments, a hygroscopic composition may be included within the catheter so that the air infusion circuit need not be serviced to replace this material.

In some embodiments of the disclosed technology, air may also be intermittently (and automatically) infused and extracted into the pressure-sensing balloon so that the balloon is in a constant state of being optimally primed, as described in further detail above. In the case of the wicking fiber or hydrophilic coating in the lumen, the air extraction may also contribute to removing and trapping any water from the air line. In the instance of a liquid-filled lumen, a hydrophilic fiber or a hydrophilic coating on the inside of the pressure lumen will provide similar benefit in allowing this lumen to handle an air bubble. In this instance, an air bubble may distort the signal, but the air water interface surface tension is defused by a hydrophilic coating in the lumen of the catheter.

Additionally, a custom extrusion and lumen shape may also be used to prevent obstruction in the case of liquid and/or air-filled lumens. In some embodiments of the technology, for example, a Foley type catheter may have a lumen that is stellate in cross sectional profile. Such a lumen is generally immune from obstruction by a water droplet, as the droplet tends to cohere to itself and push away from the hydrophobic walls. This behavior tends to disallow filling of a cross-sectional space, and allows for an air channel to remain patent around the water droplet and communicate to the sensor. The same logic applies to an air bubble in water in a hydrophilic, stellate water lumen. In this instance the hydrophilic liquid will cling to the walls and allow for a continuous water column that excludes the air bubble to the center of the lumen. The same applies for a hydrophobic liquid in a hydrophobic lumen. In some embodiments, the catheter may include an air channel, and a sensor incorporated within the catheter itself or a fluid lumen that is capable of transmitting the pressure back to a sensor.

In some embodiments, the sensing Foley catheter may include a blood pressure sensing element that may take any of several forms. In one embodiment, a blood pressure sensing element includes a pressure delivery balloon 32 (either a separate, dedicated balloon or a balloon in fluid communication with a device retention balloon or a pressure sensing balloon) that can be optically analyzed as it is inflated to determine at which pressure the vessels within the bladder or urethra are blanched and blood flow is stopped. This approach provides a reading of the perfusion pressure of the tissue abutting the pressure delivery balloon, such reading reflective of both the systemic blood pressure and vascular resistance. This embodiment of a perfusion pressure device may be used to provide early detection or monitoring of a variety of acute or emergent medical conditions such as sepsis, shock, hemorrhage, and can be particularly advantageous in detecting these conditions at an early stage. In predicting sepsis, embodiments of the invention may be capable of receiving white blood cell count information to better predict sepsis.

Other modalities may be used to detect that the tissue has been blanched or ischemic, as well, with the common methodological aspect being that of the intermittent inflation within the lumen, body cavity or bodily tissues to provide the compression of the vasculature. Embodiments of this device and associated methods may also be used to detect perfusion pressure in other areas of the body with an intermittently inflatable member and optical detection of blood flow or the presence of blood.

Tissue perfusion information may also be provided by way of sensors disposed on the shaft of the catheter such that they contact the urethral wall when the catheter is in place. These sensing technologies may include microdialysis, pyruvate, lactate, pO2, pCO2, pH, perfusion index, near-infrared spectroscopy, laser Doppler flowmetry, urethral capnography, and orthogonal polarization spectroscopy. Any of these tests may also be performed on the urine or the bladder wall itself to generate measurements of tissue perfusion.

Embodiments of a sensing Foley catheter have been used to collect data from a human subject (Figs. 7 - 9) and from a pig (Figs. 10 - 11). The human subject was a consenting and well-informed volunteer.

Fig. 7A shows an example of respiratory rate sensing data from a human subject, as provided by an embodiment of the sensing Foley catheter system. During this test period, the subject performs a respiratory sequence as follows: (1) breath being held at the end of an expiration, (2) valsalva, (3) normal respiration, (4) valsalva, and (5) breath being held at the end of an expiration. Fig. 7B shows a detailed portion of the respiratory profile of Fig. 7A, a portion of the period of normal respiration.

Fig. 8 shows an example of cardiac rate and relative cardiac output sensing data from a human subject, as provided by an embodiment of the sensing Foley catheter system, and an EKG trace as measured simultaneously and independently.

Fig. 9 shows data related to relative cardiac output sensing in a human leg raising exercise in which cardiac output increases, as demonstrated by an increased amplitude of the cardiac pulse.

The data shown in Figs. 10 and 11 were derived from studies done with Yorkshire pigs under IACUC-approved protocols. Fig. 10 shows an example of peritoneal sensing data, with a focus on respiratory rate from a pig, as provided by an embodiment of the sensing Foley catheter system. Fig. 11 shows an example of pig study that demonstrates the capability of an embodiment of the sensing Foley catheter system to detect intra-abdominal hypertension. In this study, the peritoneal cavity was accessed with a 5mm Tenamian trocar. The trocar was then attached to a 5L bag of Lactated Ringers solution via a peristaltic pump, and the solution was infused at a rate of abour1L per minute. Fluid flow was discontinued once a pressure of about 20 mmHg was obtained after which there was no net fluid flow in or out of the cavity.

Fig. 13 provides a flow diagram of an embodiment of the method of monitoring pressure as it occurs dynamically as waves of varied frequency and amplitude in the intraabdominal cavity, as detected from within the urinary bladder. Through the agency of a pressure interface, a high fidelity pressure profile is generated and transmitted proximally through a fluid column. More proximally, a pressure transducer converts the high fidelity pressure wave into a high fidelity electrical signal that is informative of pressure frequency and amplitude. The generated high fidelity electrical signal is then processed to yield data subsets that are reflective of components within the overall pressure profile, such subsets being attributable to particular physiologic sources, such as peritoneal pressure, respiratory rate, cardiac rate, relative cardiac output, and patient motion or activity.

Embodiments of the disclosed technology include a device utilizing a very small lumen for air transmission. Fig. 14 shows the pressure sensitivity using air channels with various lumen inner diameters. The readings using inner lumen diameters of 3 mm (1402), 1 mm (1404), and 0.5 mm (1406) are shown. Note that little degradation of the signal was seen when the air lumen diameter was decreased from 3mm to 1 mm and 0.5mm.

This data indicates the appropriateness of using the embodiment of the pressure transduction system in a small diameter pediatric catheter down to a size as small as 4F. Due to the lack of requirement for structural integrity that is found with the retention balloons (due to their higher pressure), the pressure lumen can easily be accommodated even in a 4F or 6F catheter that is typically provided without a retention balloon due to size constraints. In this embodiment, as well, the tip of the catheter can be lower profile than the rest of the Foley to allow for a consistently small diameter even with addition of the pressure sensing balloon. Thus, the catheter of the present invention is uniquely suited to the pediatric indication where there is a dire need for more appropriate, less invasive monitoring methods. In another embodiment, the retention balloon itself can be used as the pressure balloon, in order to minimize the number of required lumens. In one embodiment, the retention balloon is used in its fully inflated state, and is only used to track macro trends in IAP. In another embodiment, the retention balloon is only slightly inflated in order to increase balloon sensitivity to small changes in pressure. This embodiment allows for finer measurements of micro parameters, such as heart rate, relative stroke volume, relative cardiac output, respiratory rate, and relative tidal volume. A smaller pressure lumen also allows for more space in a larger catheter for other technologies, such as sensors etc.

A smaller pressure lumen also allows the tip of the catheter to be lower profile than the rest of the Foley type catheter to allow for a consistently small diameter even with addition of the pressure sensing balloon.

Embodiments of the disclosed technology may include embodiments which use the retention balloon itself as the pressure sensing balloon. This minimizes the number of required lumens allowing the overall outside diameter of the Foley type catheter to be smaller. For example, the retention balloon can be used in its fully inflated state, and used primarily to track macro trends in IAP.

Embodiments of the disclosed technology may include embodiments in which the pressure sensor is a mechanical pressure sensor, such as those using fiberoptic, strain gage, magnetic, resonant, and/or other suitable technologies.

One embodiment of the sensing Foley catheter system also includes an automated drainage line-clearing device. The drainage line is the tube that connects the Foley catheter to the drainage bag. Fig. 15 shows an embodiment for clearing the drainage line that uses a vacuum applied to the end of the drainage line. The vacuum, transmitted through the drainage line 112 and then the Foley catheter to the bladder of the patient, facilitates better draining than if the vacuum were not in place. In one aspect, the vacuum is created by a bellows 111 attached to the urine collection device or receptacle 5. The bellows 111 is expanded in its natural state, but is compressed before the urine catheter is inserted into the patient Once the catheter is in place, the bellows 111 is released, and the restoring force creates a negative pressure in the urine collection device. In another embodiment, the restoring force may also be created by a spring within the bellows 111. In another aspect, the vacuum is created by a pump. The pump may be any suitable pump, including but not limited to diaphragm pumps, peristaltic pumps, or vane pumps. The pump may be powered by a wall outlet, battery, human power, or any other suitable source. In another aspect, the vacuum is in the range of 0 to -50 mmHg.

Figs. 16A-16B, show an embodiment of the clearing mechanism comprising a device for positive airflow 113 near the start of the drainage line 11.2. Said positive airflow facilitates drainage by forcing urine to flow through the drainage line. In one aspect, shown in Fig. 16A, the positive airflow device comprises a one-way valve 115 at the end of the urine catheter that allows urine to only flow toward the urine collection device, and prevents air from entering the catheter. In another aspect, the positive airflow device comprises a diaphragm 116 attached to the start of the drainage line. Said positive airflow device also comprises a one-way valve 117 that allows air to enter the drainage line but prevents air or urine from exiting and a one way valve 118 that allows air to enter the diaphragm but prevents air from exiting. Therefore, as the diaphragm 116 is compressed, it forces air to flow through the drainage line 112. When compression is relieved, the diaphragm 116 expands into its natural state and new air is introduced through one-way valve 118. Said one-way valves 117 and 118 could be any suitable valves, including but not limited to umbrella valves and duckbill valves. In another aspect, shown in Fig. 16B, the diaphragm 121 is not located at the start of the drainage line 112, but is connected to the start of said drainage line through a lumen 123 or tube that runs from the start of the drainage line to the diaphragm 121. The diaphragm 121 also comprises a one-way valve 127 that allows air to enter the drainage line but prevents air or urine from exiting and a one way valve 125 that allows air to enter the diaphragm but prevents air from exiting. In yet another aspect (not shown), the positive airflow device comprises a pump. The pump may be any suitable pump, including but not limited to a diaphragm pump, peristaltic pump, or vane pump. The pump may be powered by a wall outlet, battery, human power, or any other suitable source. In yet another aspect, the positive airflow device comprises a syringe attached to 5 the drainage tube. The syringe may attach to the drainage tube with a luer lock, septum valve, or any other suitable interface.

In another embodiment, the clearing mechanism comprises a coating on the inside of the drainage tube to reduce surface tension and facilitate drainage. In one aspect, said coating is a hydrophobic polymer, including but not limited to PTFE or FEP.

In yet another embodiment, the clearing mechanism comprises a tubular hydrophobic vent filter (not shown) that can be inserted into the drainage lumen of the device such that air will be evacuated throughout its length. A segmental hydrophobic vent can also be incorporated at set intervals to ensure that air is evacuated from the tube as it passes these regions. While others have attempted to prevent air locks with a hydrophobic vent filter at the interface of the Foley catheter and drainage tube, this approach still results in air locks regularly if the vent is not at the zenith of the drainage tube and pointed downward (such that the drainage tube end of the vent is below the Foley catheter side). In the preferred design the hydrophobic vent will be interspaced at minimum of 1-2 foot intervals to prevent submersion of the vents in urine (a problem that found with the currently-used urinary catheter which is vented only at the Foley adapter). By providing redundancy the present invention prevents the failure of the vent due to submersion since all of the intermittent vents would have to be submerged which is not possible, based on our bench top tests with a redundant loop. In the ideal configuration the vent will be a PTFE or ePTFE material and will be affixed with a barb and or grommetted into the tube at intervals to allow for easy manufacturability. In an alternative embodiment, the vent takes the form of a slit or spiral that runs the length of the drainage tube, thereby allowing air to escape the tube at any point. This prevents the drainage tube from being positionally dependent when preventing and/or eliminating airlocks. Fig. 39A shows an example of a drainage tube with a slit vent 272, and Fig. 39B shows an example of a drainage tube with a spiral vent 273.

In an alternative embodiment, air locks are prevented by means of an extendable drainage tube (not shown), which prevents pockets of air from forming in the high portions of the tube and urine from gathering in the low portions. An extendable tube prevents this from occurring by keeping the tube as straight as possible between the urinary catheter and the collection bag. In one aspect, the extendable drainage tube is composed of multiple telescopic sections that can be extended or collapsed to match the distance from the patient to the collection bag. In another aspect, the drainage tube is pleated to form an accordion, which can be extended or collapsed as necessary. In yet another aspect, the tube is coiled. In yet another aspect, the drainage tube is retractable by means of a spring coil that wraps the tubing around a wheel to achieve the appropriate length.

In another embodiment, the clearing mechanism comprises a tube with an inner diameter less than 0.25 inches as the drainage tube (not shown), such that no air pockets are able to move up the length of the tube. This is possible due to the surface tension within the smaller tubes, which prevent movement of fluid when one end of the tube is closed to atmosphere (as in the case of the bladder). Thus, the drainage tube always remains full of urine, and for each volume of urine produced the same volume of urine must exit the drainage tube, as urine is incompressible. In another embodiment, the inner diameter is less than 0.125 inches. In another aspect, said drainage tube acts as a siphon and provides a small, safe amount of vacuum to the bladder.

The use of small-diameter tubing also results in a smaller volume of residual urine in the drainage tube compared with the prior art. Having a smaller residual volume is preferential, as it allows urine to move more quickly from the patient's bladder to the collection vessel. The speed of this transport is important in order to take measurements of the urine that has been produced more recently. This is particularly important for patients with low rates of urine production, as it takes their urine even longer to be transported from the bladder to the collection vessel. For example, for a patient producing only 10 mL/hr of urine with a standard drainage tube (around 40 mL residual volume), measurements of their urine in the collection vessel will lag true urine production by 4 hours. By contrast, with smaller tubing (such as tubing having around 5 mL residual volume), measurements will only lag true production by 30 minutes.

In another embodiment, shown in Fig. 17, the clearing mechanism comprises an apparatus for automated massaging, or squeezing, of the drainage line 112. In one aspect, the squeezing apparatus comprises a peristaltic pump 129. Said peristaltic pump 129 also provides slight vacuum to the bladder, which helps to facilitate drainage as described herein. In another aspect, the squeezing mechanism comprises a slider-crank mechanism attached to a rotary motor. In another aspect, the squeezing mechanism comprises a solenoid. In another aspect, the clearing mechanism further comprises one-way valves on either side of the squeezing mechanism to force urine and air to only flow down the tube and further provide vacuum to the bladder.

In another embodiment, air locks are removed through use of a pulsatile mechanical, vibratory acoustic, thermal, or electromagnetic stimulus that results in movement of the drainage tubing and/or the fluid within. This vibration, in combination with the pressure gradient driving the urine preferentially from the patient to the urine drainage bag, allows the urine to move forward in small increments until the resistance of the air lock has been overcome. At this point, a siphon is created and normal drainage can resume. The pulsatile stimulus is effective due to the hysteresis involved in the flow of the urine in the presence of a pressure gradient. Small movements of the urine due to energy pulses will have a net effect of moving the urine away from the patient. In one aspect using pulsatile energy, a vibratory stimulus is employed. The vibratory stimulus described can be created using a coin vibration motor, eccentric motor, or other similar means.

As an alternative to the vibratory stimulus, the drainage tube may be pinched or rolled intermittently, which has a similar net effect of moving the urine away from the patient due to hysteresis. This pinching or rolling may be achieved using a peristaltic-like mechanism, slider-crank mechanism, or other similar means. An alternative approach would be to use a pneumatic or hydraulic pump to cycle compression and decompression, like a sphygomomanometer, on different sections of the tube to mimic manual milking of the tube. This approach is distinct from the automated massaging or squeezing described above, in that only a slight pulse of stimulus is required. The pulsatile approach, then, can avoid generating vacuum in the bladder, which may adversely affect bladder tissue. The vibratory or pinching stimulus may be placed near the patient, near the drainage tube, or anywhere in between.

In another aspect using pulsatile energy, an acoustic stimulus is employed. The acoustic stimulus may be of a subsonic frequency designed to agitate the fluid but not the patient (due to the stimulus being below the range of hearing). The stimulus may also be in the sonic range or even in the supersonic range to achieve higher energy delivery. In 5 the acoustic embodiment, the pressure waves will be transmitted down the fluid column generating the same hysteresis effect.

In another aspect using pulsatile energy, an electromagnetic stimulus is employed. The electromagnetic stimulus may be a cuff or other device external to the drainage tube that creates pulses of electromagnetic energy. This energy has an effect on the salts in the urine, effectively agitating it slightly toward the drainage bag. The principles underlying this method are that of an electromagnetic pump, which is used in other applications. The electromagnetic approach takes advantage of the same hysteresis effect as the other approaches, and has the same effect of removing air locks by agitating the urine toward the drainage back until a siphon effect is achieved.

In another aspect using pulsatile energy, a thermal stimulus is employed. The thermal stimulus may be used to rapidly heat and cool a small portion of the drainage tubing, thereby expanding and contracting the urine or air within. In the expansion phase, the leading edge of the urine or air preferentially expands toward the drainage bag, due to the pressure gradient. Similarly, in the contraction phase, the tailing edge of the urine or air moves toward the drainage bag. The thermal stimulus thus takes advantage of the same hysteresis effect as the other approaches. Rapid heating of the urine or air can be achieved with a heating coil, chemical reaction, or other similar means, while rapid cooling of the urine or air can be achieved with a Peltier cooler, chemical reaction, gas expansion, or other similar means.

In another embodiment the mechanical, acoustic, electromagnetic, thermal, vibratory or pinching stimulus may be continuous, scheduled, or sensor-based. In the continuous embodiment, the stimulus is always on. In the scheduled embodiment, the stimulus repeats itself after a given time period, such as, but not limited to, every 1 minute, 5 minutes, 10 minutes, 30 minutes, or 1 hour. In the sensor-based embodiment, the mechanical, acoustic, electromagnetic, thermal, vibratory or pinching stimulus is applied whenever an air lock is suspected or detected based on urine output and sensed pressures. This detection can be accomplished in a variety of ways, including, but not limited to, a flow sensor, an optical sensor that distinguishes between urine and air, or an in-line oxygen sensor. Furthermore, each of these embodiments could be expected to interfere with pressure measurements in the sample collection vessel described below and will preferably be performed immediately after 5 a siphon activation to allow for minimization of the risk of missing a vessel emptying or interfering with a specific gravity measurement

Fig. 18 shows another embodiment of the pinching or rolling stimulus, the lumens are compressed sequentially by rollers 131 such that they are never all compressed at the same time. This feature serves to prevent all lumens from becoming obstructed, a scenario that could cause urine to back up in the patient's bladder and lead to detrimental conditions. Having multiple lumens that are only compressed one at a time also helps reduce the amount of negative pressure that is applied to the bladder wall. This prevents trauma to the soft tissues. In one aspect, the lumens lay side-by-side in a strip fashion, and the pinching or rolling mechanisms are offset such that they can only compress one lumen at a time.

Preferably, an entire drain tube will be cleared with one roll; at a minimum, one half of a drain tube height should be cleared, given a maximum air lock height. Advantageously, these rollers can handle high viscosity urine. The rollers comprise cam profiles that may be round or oval-which can provide varying pressure for clearing clots. Should a blood clot obstruction occur at a Foley catheter inlet hole, the rollers can be used to temporarily reverse the flow of urine to dislodge the clot, or (as previously described) intentional vibration of the fluid column can be used to dislodge the clot. The roller position can be selectively controlled so as to avoid "parking" on tubes. This ensures that flow is completely unobstructed from the bladder to the drainage bag. Controlling the parked location can be accomplished with any suitable means, including, but not limited to a stepper motor, current sensing of the motor (current will drop when the rollers are not compressing the tubes), a limit switch, an encoder, magnetic positioning, detection of a change in tube diameter as it is compressed, and/or pressure sensors on the lumen or roller. However, in certain instances, parking the rollers on the tubing may be beneficial for selectively limiting the flow if it is too high for the chamber to handle, particularly when first intubating the bladder. In these instances, selective control of the roller position will be used to ensure one of the tubes is compressed. The rollers can be activated manually, using a timed means, or automatically triggered if, based on the number or urine drips in a chamber, no urine output is detected for a specified number of minutes. Suction trauma to the soft tissues is prevented by setting the roller speed is set so that is occurs slowly enough to remain quasi-static. In the event 5 of an air lock with an empty bladder, for example, in one embodiment the roller would pull gentle suction on one tube, but the suction transmitted to the bladder would be limited by the ability of fluid to move from one tube to the other by virtue of their being joined at the proximal end of the tube where it connects to the Foley catheter.

Fig. 19 shows another embodiment comprising multiple lumens 145 organized circumferentially around a stiff member 141 that the pinching or rolling mechanism 143 rotates around, thereby compressing one lumen at a time and avoiding complete obstruction of all lumens. Fig. 20 shows an alternative embodiment in which the lumens 145 are organized such that they can only be completely compressed when pinched in a certain direction 147, or 148. A plurality of rolling or pinching mechanisms are used to compress the tube sequentially from multiple directions, and each mechanism can only compress those lumens that are designed to be compressed in that direction. Fig. 20 illustrates an example of lumen geometries that are only fully compressed in a preferential direction. In the non20 preferential direction, the lumens cannot be completely compressed. In this example, lumens 147 will be compressed with the illustrated pinching force, while lumens 148 will not. Alternatively, a single rolling or pinching mechanism rotates around the tube to compress it sequentially from multiple directions. In another embodiment of the sequential pinching or rolling stimulus, the portion of the tube that is pinched or rolled is only a small portion of the entire drainage tube, such that the geometry of the rest of the drainage tube is not limited to the geometries required to facilitate sequential compression of the lumens. In another embodiment of the peristaltic pumps used for massaging, squeezing, or pulsing, the pump is a finger-style peristaltic pump that uses linear motion to stimulate the drainage tubing.

In another embodiment, a pressure sensing lumen may be incorporated into the tubing to allow for measurement of pressure within the drain tube, Foley catheter or bladder itself. This pressure measurement can be used to control the pump or line clearing mechanism to allow for effective air lock removal without the generation of negative pressure and suction trauma in the bladder. This device may also be used in combination with a pressure sensing Foley catheter. This combination will allow for the effective measurement of true bladder pressure and activation of the pump to ensure that the sensed bladder pressure is truly a result of intra-abdominal hypertension and not the result of a confounding air lock.

The sensing balloon of the Foley can also be incorporated proximally into the Foley catheter or be attached to the drainage tube in order to minimize the intravesical profile of the device. The sensing lumen could also be another lumen in the tube that conducts the pressure through the lumen to the pressure sensor and roller pump. In the absence of an air lock, the pressure seen in fluid communication with the inside of the bladder is actually a vacuum. In order to provide an accurate measurement of bladder pressure in the setting of a siphon effect (i.e. with a vented Foley drain system or in the absence of any air lock) the pumping mechanism can actually be driven backwards until it has offset the siphon effect. There will still be no net movement of fluid in this scenario and the pump action will be increased until further increases do not generate an increase in sensed pressure. At this point the true bladder pressure can be read and the flow from the bladder can be allowed to resume.

Fig. 21 shows a graph of the pressure profile, pressure (mmHg) 149 over time (seconds) 151 in the drain tube while the peristaltic roller pump is activated. The graph shows an airlock being formed and pressure building 153, vacuum generated in drainage tube/Foley catheter by peristaltic action of pump and detected by pressure sensor 155, elimination of airlock with the pump parked on one tube 157, and airlock eliminated with the pump parked on none of the tubes 159. No matter how the vacuum is generated (peristaltic pump, integrated gear pump, etc.) the bladder is at risk of suction trauma. This suction trauma can cause mucosal irritation and bleeding and can increase the risk of bladder infection. Monitoring the pressure and activating/deactivating pump operation based on the sensed pressure mitigates this risk and allows for effective line clearance without exposing the bladder to excessive vacuum. In addition, in the event that a siphon effect is generated, purposefully occluding one of the outflow tubes can decrease the overall vacuum generated within the bladder. Temporarily reversing the action of the pump can offset the siphon and provide a true bladder pressure.

Fig. 22 is a table comparing IAP measurements using a standard drainage line and IAP sensor with the present invention in combination with a pressure-sensing Foley catheter under air lock 161 and siphon 163 effects. A sheep bladder was used to compare pressure measurements between standard drainage technologies and the present invention. In the presence of an air lock, traditional technologies to measure IAP report false positive values, whereas the Accuryn device shows greater accuracy. In the absence of an air lock, but in the presence of a siphon (due to a full drainage tube), the traditional technology reports accurate values if used intermittently, with a valve in place to temporarily block flow from the bladder to the drainage tube. The present device also reports accurate values in the presence of a siphon. However, when used continuously without a valve, the traditional technology severely underreports the true pressure. Without air lock prevention and elimination, IAP cannot be accurately and reliably measured. In addition, respiratory rate, tidal volume, heart rate, cardiac output and stroke volume readings from the bladder may be diminished and/or corrupted due to the floating baseline of pressure within the bladder.

In yet another embodiment (not shown), the present invention and the pressure-sensing Foley catheter can be used together to detect and clear obstructions from blood clots or other obstructions. During milking of the drainage tube, if the pressure in the drainage tube spikes while the pressure within the bladder remains unchanged, this is indicative of a blockage between the bladder and the termination of the pressure sensing lumen. To clear this blockage, additional negative pressure can be generated using the massaging rollers until the pressure suddenly drops and matches the pressure within the bladder. This is indicative that the blockage has been cleared. In yet another embodiment, blockages such as those from blood clots can be prevented by ensuring that the inner diameter of the drainage lumen/tube only gets larger or remains the same size from the bladder to the drainage bag. When the opposite occurs, this creates the potential for bottlenecks that can become a site for obstruction.

Figs. 23A and 23B show another embodiment of the disclosed technology which allows for a smaller profile catheter, particularly in the area of the pressure balloon. In this embodiment retention balloon 2302 is proximal to pressure balloon 2304. The catheter shaft has a reduced diameter area 2306 below pressure balloon 2304. Reduced area 2306 allows the pressure balloon to reduce to a smaller diameter when it is deflated, as shown in Fig. 15B. Reduced diameter area 2306 may be formed by stepping down the outer diameter of the catheter lumen, or by cutting away part, or all, of the outer surface of the catheter outer lumen, or by using an inner lumen within the outer catheter shaft.

Figs. 24 show the placement of an exemplary embodiment of preperitoneal sensing implant. Implantable embodiments may employ a balloon 101 positioned in the pre- peritoneal space.

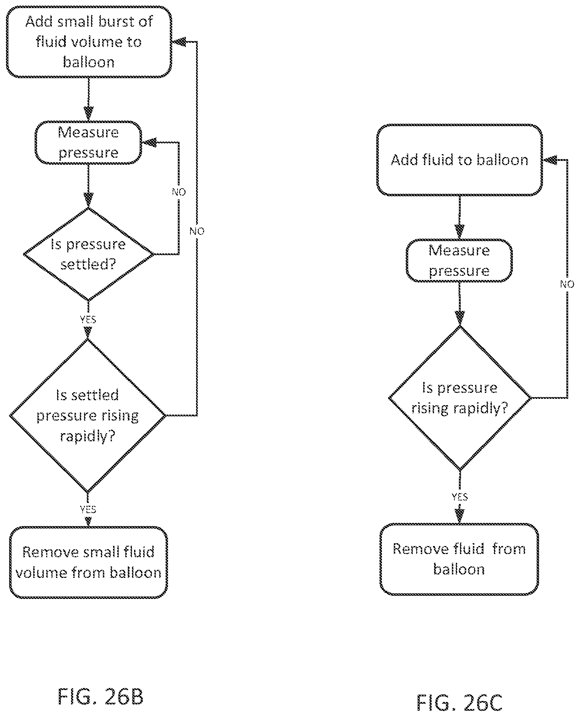

Fig. 25A shows a graph representing a pressure balloon priming method in some embodiments. Here, small volume bursts (roughly about 0.3 cc) of fluid volume are added to the pressure sensing balloon and the pressure within the balloon is measured. Small volume bursts of fluid are introduced until the measured pressure within the balloon settles to a stable pressure 2501. This transition is shown at inflection point 2502. Volume bursts are introduced past this point until the measured pressure starts to rapidly increase (for example if slope 2504 of the curve is greater than about 2mmHg/10ms). This inflection point is shown at 2504. At this point the pressure within the balloon is reduced to a pressure around or slightly above stable pressure 2501. This pressure represents the prime pressure measuring pressure in some embodiments. This process is also represented in the flowchart in Fig. 26B.

The small volume bursts of fluid may be from around 0.2cc to around 0.4cc. The small volume bursts of fluid may be from around 0.1cc to around 0.5cc. The small volume bursts of fluid may be up to around 0.5cc. The small volume bursts of fluid may be up to around 1.0cc.

Fig. 25B shows a graph representing a pressure balloon priming method in some embodiments. This method is similar to that shown in Fig. 25A, except that the pressure is increased within the pressure sensing balloon more smoothly, without the bursts shown in Fig. 25A. Fluid volume is added to the pressure sensing balloon and the pressure within the balloon is measured. Balloon pressure is increased until the measured pressure within the balloon settles to stable pressure 2505. This transition is shown at inflection point 2506. Balloon pressure is increased past this point until the measured pressure starts to rapidly increase (for example if slope 2510 of the curve is greater than about 2mmHg/10ms). This inflection point is shown at 2508. At this point the pressure within the balloon is reduced to a pressure around or slightly above stable pressure 2505. This pressure represents the prime pressure measuring pressure in some embodiments. This process is also represented in the flowchart in Fig. 26C.

Fig. 26A shows a flowchart of the balloon priming process of certain embodiments of the invention. Embodiments of the disclosed system and method include automatic pressure tuning by a controller. Accordingly, the tuning system can detect the optimum target pressure and volume to inflate the balloon by monitoring sensed pressure signals and adding or removing air volume as needed. For example, upon insertion of the catheter, a pressure tuning circuit that regulates the balloon volume and pressure will inflate the balloon until it detects a physiologic-sourced pressure rate. Upon sensing that rate, the pressure tuning controller will add or subtract minute amounts of air or fluid (roughly about 0.3 cc) in a routinized sequence until the amplitude of the sensed wave is greatest. The control feedback loop between the optimally tuned pressure (manifesting as balloon pressure and volume) and the sensed physiologic pressure profile iterates continuously and or as needed to ensure high fidelity measurement of the physiologic data. In some embodiments, automatic pressure tuning may be performed in the apparent background while the physiologic data is being transmitted and displayed; in other embodiments the system may suspend transmission of physiologic data during a pressure tuning sequence.

The minute amounts of air or fluid may be from around 0.2cc to around 0.4cc. The minute amounts of air or fluid may be from around 0.1cc to around 0.5cc. The minute amounts of air or fluid may be up to around 0.5cc. The minute amounts of air or fluid may be up to around 1.0 cc.

Fig. 27A and 27B show an embodiment of the invention which includes a fiber optic pressure sensor. Fig. 27A shows a cutaway view of a catheter tip which encases a fiber optic pressure sensor. In this embodiment, catheter tip 2702 includes 2 lumens 2704 and 2706. Lumen 2704 in this embodiment is a drainage lumen and lumen 2706 is a dedicated fiber optic lumen which includes fiber optic sensor. Fiber optic sensor includes fiber optic fiber 2708 and fiber optic sensor tip 2710. Although the fiber optic sensor is shown here in a dedicated lumen, the sensor may alternatively be in the drainage lumen. Sensor hole 2712 allows the fiber optic sensor to be in fluid communication with the fluid in the bladder and exposes fiber optic sensor tip to the pressures in the bladder. The diameter of fiber optic cable 2708 is around .004" and the diameter of sensor tip 2710 is around .010". The diameter of the tip of the catheter in this embodiment is around 16 Fr. Or around .210".

Fig. 27B shows an outside view of the tip of a catheter which encases a fiber optic pressure sensor. Retention balloon 2714 is attached to the catheter near catheter tip 2702. Urine drainage hole 2716 is distal to retention balloon 2714. Sensor hole 2712 may be distal or proximal to the urine drainage hole, or may be the same as the drainage hole and is shown distal to the retention balloon. Note that the fiber optic pressure sensor is encased inside the catheter and cannot be seen here. The fiber optic pressure sensor runs from the tip of the catheter back to the proximal end of the catheter and may terminate at a controller.

Although Figs. 27A and 27B show a fiber optic pressure sensor, any appropriate pressure sensor technology could be used.

Fig. 28 shows an embodiment of the invention with more than one pressure lumen. This embodiment is similar to that shown in Fig. 6. Fig. 28 shows an embodiment with more than one sensing lumen. Sensing lumens 2806 may be pressure sensing lumens only or may sense analytes and/or take other measurements. Retention balloon lumen 2802 and urine lumen 2804 are also shown. The advantage of more than one sensing lumen is to identify and filter out noise. Pressure, or other, measurements are detected through both lumens 2806. Assuming proper calibration, the real signals through the two lumens are generally similar over time. However, if one of the signals shifts or becomes noisy, while the other signal does not, it can be assumed that the shifting and/or noisy signal is in fact noise, and/or an artifact, and not reflective of an anatomical measurement. By having more than one sensing lumen, and continually comparing the two or more signals, the controller can identify and filter out potentially noisy signals, allowing for more accurate measurements. The additional one or more lumens may merge at any location along the catheter length, or the lumens may remain separate the full length of the catheter, to the catheter tip. Preferably in this embodiment, more than one sensing lumens terminates at a single sensor. For example, two pressure sensing lumens may terminate in one pressure sensing balloon at or near the tip of the catheter, as is shown in Fig. 28. However, it would also be possible to have each lumen terminate at its own sensor and/or sensing balloon.

Figs. 29A - 29C show an embodiment of the invention where the pressure sensor is in fluid communication with the urine lumen of a Foley catheter, but may reside outside of the bladder. Fig. 29A shows fluid chamber 2902 with port 2904. Port 2904 is connected to the urine drainage lumen of a Foley type catheter which allows the interior/receiving channel of fluid chamber 2902 to fill with urine. Pressure sensing balloon 2906 is contained inside fluid chamber 2902 and is in fluid communication with pressure line 2908. Pressure sensing balloon 2906 and pressure line 2908 are filled with fluid, either a gas or a liquid. Pressure line 2908 is connected to a pressure sensor such as a pressure transducer. This embodiment allows the pressure sensing balloon to reside outside of the bladder, and to be connected, managed, cleaned, maintained and disconnected while the Foley type catheter is in place in the bladder. In addition, this embodiment of the invention allows the pressure sensor to be used with any Foley type catheter.

Fig. 29A shows pressure sensing balloon 2906, but the pressure sensor can be any kind of pressure sensor including a mechanical or fiber-optic pressure sensor. Priming of pressure sensing balloon 2906 may be done using any of the methods mentioned herein.

Fig. 29B shows a Foley type catheter with retention balloon 2910, urine drainage opening 2912 which is in fluid communication with the urine drainage lumen. Retention balloon port 2914 and urine drainage port 2916 are at the proximal end of the catheter. Secondary urine lumen port 2918 may connect to the urine drainage lumen at any point along the length of the catheter. Urine lumen port 2918 may be connected to fluid chamber port 2902 shown in Fig. 29A so that pressure sensing balloon 2906 is in fluid communication with urine in the urine lumen of the Foley type catheter and ultimately, with the urine in the bladder. Pressure measurements can be taken over time via port 2918 and analyzed in any of the ways disclosed herein. To improve pressure measurements, drainage port 2916 may be periodically closed or blocked. Blocking of drainage port 2916 may be done mechanically, with a stopcock or valve, or automatically, for example with a solenoid valve connected to, and controlled by, the controller mentioned in some embodiments herein.

Fig. 29C shows a standard Foley type catheter which is connected to adapter 2920. Adapter 2920 can be connected to urine drainage port 2916. Adapter 2920 has two ports, urine drainage port 2922 and secondary urine lumen port 2924. Urine lumen port 2918 may be connected to fluid chamber port 2902 shown in Fig. 29A so that pressure sensing balloon 2906 is in fluid communication with urine in the urine lumen of the Foley type catheter and ultimately, with the urine in the bladder. Pressure measurements can be taken over time via port 2918 and analyzed in any of the ways disclosed herein. To improve pressure measurements, drainage port 2916 may be periodically closed or blocked. Blocking of drainage port 2916 may be done mechanically, with a stopcock or valve, or automatically, for example with a solenoid valve connected to the controller. An advantage of this embodiment is that adapter 2920 can be used with any Foley type catheter to measure pressure. In addition, adapter 2920 can be attached to and removed from a Foley type catheter after the Foley type catheter is already in place in the patient's bladder.

Figs. 30A- 30B show an embodiment of the invention where the pressure sensor is in fluid communication with the urine lumen of a Foley catheter, but may reside on a separate catheter. Foley type catheter 3002 is shown with urine lumen 3004 and urine drainage opening 3006. Small pressure sensing catheter 3008 with pressure sensing balloon 3010 is shown inside the urine drainage lumen of the Foley type catheter. The outer diameter of the pressure sensing catheter is small enough so that it fits within the urine drainage lumen of a Foley type catheter. For example the outer diameter of the pressure sensing catheter may be less than about 4mm, alternatively the outer diameter of the pressure sensing catheter may be less than about 3mm, alternatively the outer diameter of the pressure sensing catheter may be less than about 2mm, alternatively the outer diameter of the pressure sensing catheter may be less than about 1 mm.

The pressure sensor on the pressure sensing catheter may be near the distal end of the pressure sensing catheter, or it may be anywhere along the length of the catheter. The pressure sensor may be a pressure sensing balloon, or it may be any type of pressure sensor. In the case of a pressure sensing balloon, the inflated balloon may be smaller than the inner diameter of the urine drainage lumen of the Foley type catheter, or the inflated balloon may be large enough to fill the urine drainage lumen of the Foley type catheter.

The inflated pressure sensing balloon may fill the urine drainage lumen of the Foley type catheter allowing for better pressure measurements. The pressure sensing balloon may be periodically deflated or partially deflated to allow urine to flow from the bladder through the Foley type catheter. The controlling of the pressure sensing balloon inflation cycle may be controlled by the controller of the present invention.

The outer diameter of the inflated pressure sensing balloon may less be than about 5mm, alternatively the outer diameter of the pressure sensing catheter may be less than about 4mm, alternatively the outer diameter of the pressure sensing catheter may be less than about 3mm, alternatively the outer diameter of the pressure sensing catheter may be less than about 2mm, alternatively the outer diameter of the pressure sensing catheter may be less than about 1mm.

Fig. 30B shows a standard Foley type catheter with retention balloon 3012, urine drainage opening 3006, retention balloon port 3014, and urine drainage port 3016. Adapter 3018 is shown connected to urine drainage port 3016. Adapter 3018 has two ports, urine drainage port 3020 and secondary urine lumen port 3022. Pressure sensing catheter 3008 is shown in urine lumen port 3022. In this way the pressure sensing catheter is in fluid communication with the urine drainage lumen of the Foley type catheter. Proximal end of pressure sensing catheter 3008 is connected to a pressure sensor such as a pressure transducer, similar to other embodiments herein. Pressure sensing catheter 3008 may have only a single lumen, the sensing balloon lumen, or it may contain other lumens. In the case where the pressure sensor of the pressure sensing catheter is a mechanical pressure sensor, the pressure sensing catheter may have no lumens, or the pressure sensing catheter may have a balloon for sealing the urine drainage lumen of the Foley type catheter.

Pressure measurements can be taken over time using the pressure sensing catheter and analyzed in any of the ways disclosed herein. To improve pressure measurements, drainage port 3020 may be periodically closed or blocked. Blocking of drainage port 3020 may be done mechanically, with a stopcock or valve, or automatically, for example with a solenoid valve connected to the controller. An advantage of this embodiment is that pressure sensing catheter 3008 can be used with any Foley type catheter to measure pressure. In addition, pressure sensing catheter 3008 can be inserted and removed from a Foley type catheter after the Foley type catheter is already in place in the patient's bladder.

Fig. 31 shows another embodiment of the invention where a retention balloon is not present, for example, in a chest drainage tube. Shown here are fluid drainage holes 3102 and pressure balloon 3104. Drainage holes are shown here both proximal to, and distal to, pressure balloon 3104, however the drainage holes may be only distal to, or only proximal to, the pressure balloon. Multiple drainage holes are shown here, but in some embodiments only one drainage hole may exist.

Pressure balloon port hole 3106 is in communication with the pressure fluid lumen which is in fluid communication with pressure line 3108. Fluid drainage line 3110 is in fluid communication with the one or more fluid drainage holes 3102.

As described herein, pressure line 3108 is in fluid communication with a pressure transducer or other type of pressure sensor.

Fluid drainage line 3110 may be used with any of the clearing mechanisms described herein. For example, a rolling mechanism, similar to that shown in Fig. 18, may be used to help clear fluid from the chest or other body cavity. In the case where rollers are used to help clear the chest, pressure measurements may show a pressure wave related to the roller action when the fluid drainage line is clearing adequately. A flattening of the roller related pressure wave may indicate that the drainage line is not draining adequately and may be an indication of a clot or other blockage somewhere in the drainage tube and/or drainage line, including possibly at a drainage hole of the drainage catheter. If such a flattening of the pressure wave is detected, the rollers may be programmed to reverse direction, either manually or automatically, causing fluid to temporarily flow toward the chest cavity rather than away from the chest cavity. This action may serve to dislodge the blockage and allow fluid again to flow adequately through the drainage line. Other actions may be taken to attempt to clear the drainage line, including flushing the drainage line, mechanically unblocking the drainage line etc.

By monitoring the pressure within the chest cavity, or other body cavity, fluid drainage may be monitored and action taken if drainage is not adequate. For example, in addition to a flattening of the pressure wave described above, a sustained increase of pressure within the body cavity may be an indication that fluid drainage is not adequate. A sustained decrease in pressure within the body cavity may be an indication that fluid drainage is no longer necessary.

A pressure sensing balloon is shown here, but any suitable type of pressure sensor may be used.

In the case of a chest drainage tube, a retention balloon is not necessary because the chest tube is likely sutured or otherwise fixed to the outer chest wall after insertion. This may also be the case for other types of drainage tubes, such as a wound drainage tube. The pressure sensing balloon/mechanism may sense anatomical pressures to determine anatomical information such as peritoneal pressure, respiratory rate, and cardiac rate. In addition or alternatively, the pressure sensing balloon/mechanism may sense the presence of clots, or other blockages which prevent the drainage tube from draining adequately.

In another embodiment, a physical filter may be used at any location along the length of a sensing lumen. For example, a filter may be placed between a pressure sensing lumen and a pressure transducer. A filter may remove a signal offset allowing a more sensitive sensor to be used. A filter may be made of any suitable material, such as polymer foam.

Any of the priming protocols disclosed here, or any combination thereof may be used in any of the embodiments of the invention.

Although the pressure sensing balloon and/or sensor is shown distal to the retention balloon in some of the figures herein, the pressure sensing balloon and/or sensor may also be proximal to the retention balloon.

Embodiments of the invention include a pressure sensing balloon incorporated into a chest tube or breathing tube to monitor pressure in the lungs and/or chest. Similar to other embodiments disclosed herein, a pump, vacuum, roller device or other technology may be used to help clear the chest tube of fluids and/or other blockages. Chest flow fluid volume (gas and/or liquid) may be measured using technologies disclosed herein.

### Example of Data Processing System

Fig. 32 is a block diagram of a data processing system, which may be used with any embodiment of the invention. For example, the system 3200 may be used as part of a controller. Note that while Fig. 32 illustrates various components of a computer system, it is not intended to represent any particular architecture or manner of interconnecting the components; as such details are not germane to the present invention. It will also be appreciated that network computers, handheld computers, mobile devices, tablets, cell phones and other data processing systems which have fewer components or perhaps more components may also be used with the present invention.

As shown in Fig. 32, the computer system 3200, which is a form of a data processing system, includes a bus or interconnect 3202 which is coupled to one or more microprocessors 3203 and a ROM 3207, a volatile RAM 3205, and a non-volatile memory 3206. The microprocessor 3203 is coupled to cache memory 3204. The bus 3202 interconnects these various components together and also interconnects these components 3203, 3207, 3205, and 3206 to a display controller and display device 3208, as well as to input/output (I/O) devices 3210, which may be mice, keyboards, modems, network interfaces, printers, and other devices which are well-known in the art.

Typically, the input/output devices 3210 are coupled to the system through input/output controllers 3209. The volatile RAM 3205 is typically implemented as dynamic RAM (DRAM) which requires power continuously in order to refresh or maintain the data in the memory. The non-volatile memory 3206 is typically a magnetic hard drive, a magnetic optical drive, an optical drive, or a DVD RAM or other type of memory system which maintains data even after power is removed from the system. Typically, the non-volatile memory will also be a random access memory, although this is not required.

While Fig. 32 shows that the non-volatile memory is a local device coupled directly to the rest of the components in the data processing system, the present invention may utilize a non-volatile memory which is remote from the system; such as, a network storage device which is coupled to the data processing system through a network interface such as a modem or Ethernet interface. The bus 3202 may include one or more buses connected to each other through various bridges, controllers, and/or adapters, as is well-known in the art. In one embodiment, the I/O controller 3209 includes a USB (Universal Serial Bus) adapter for controlling USB peripherals. Alternatively, I/O controller 3209 may include an TEEE-1394 adapter, also known as FireWire adapter, for controlling FireWire devices.

Some portions of the preceding detailed descriptions have been presented in terms of algorithms and symbolic representations of operations on data bits within a computer memory. These algorithmic descriptions and representations are the ways used by those skilled in the data processing arts to most effectively convey the substance of their work to others skilled in the art An algorithm is here, and generally, conceived to be a self-consistent sequence of operations leading to a desired result The operations are those requiring physical manipulations of physical quantities.

It should be borne in mind, however, that all of these and similar terms are to be associated with the appropriate physical quantities and are merely convenient labels applied to these quantities. Unless specifically stated otherwise as apparent from the above discussion, it is appreciated that throughout the description, discussions utilizing terms such as those set forth in the claims below, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

The techniques shown in the figures can be implemented using code and data stored and executed on one or more electronic devices. Such electronic devices store and communicate (internally and/or with other electronic devices over a network) code and data using computer-readable media, such as non-transitory computer-readable storage media (e.g., magnetic disks; optical disks; random access memory; read only memory; flash memory devices; phase-change memory) and transitory computer-readable transmission media (e.g., electrical, optical, acoustical or other form of propagated signals-such as carrier waves, infrared signals, digital signals).

The processes or methods depicted in the preceding figures may be performed by processing logic that comprises hardware (e.g. circuitry, dedicated logic, etc.), firmware, software (e.g., embodied on a non-transitory computer readable medium), or a combination of both. Although the processes or methods are described above in terms of some sequential operations, it should be appreciated that some of the operations described may be performed in a different order. Moreover, some operations may be performed in parallel rather than sequentially.

Unless defined otherwise, all technical terms used herein have the same meanings as commonly understood by one of ordinary skill in the medical arts. Specific methods, devices, and materials are described in this application, but any methods and materials similar or equivalent to those described herein can be used in the practice of the present invention. While embodiments of the invention have been described in some detail and by way of illustrations, such illustrations are for purposes of clarity of understanding only, and are not intended to be limiting. Various terms have been used in the description to convey an understanding of the invention; it will be understood that the meaning of these various terms extends to common linguistic or grammatical variations thereof. Further, while some theoretical considerations may have been advanced in furtherance of providing an understanding of the technology, the appended claims to the invention are not bound by such theory. Moreover, any one or more features of any embodiment of the invention can be combined with any one or more other features of any other embodiment of the invention, without departing from the scope of the invention. Still further, it should be understood that the invention is not limited to the embodiments that have been set forth for purposes of exemplification, but is to be defined only by a fair reading of claims appended to the patent application, including the full range of equivalency to which each element thereof is entitled.

The below statements numbered 1-46 correspond to the claims of the PCT parent application of this divisional application as filed. They are not the claims of this divisional application.
1. A fluid pressure sensing assembly, comprising:
   a catheter having a length and an expandable retention member located near or at a distal end of the catheter, the catheter defining a drainage lumen at least partially through the catheter length such that a distal end of the drainage lumen terminates at a drainage opening defined near or at the distal end of the catheter;
   a fluid chamber defining a receiving channel and a port fluidly coupled to the drainage lumen such that the receiving channel is in fluid communication with the drainage opening; and
   a pressure sensing mechanism located within the fluid chamber, wherein a fluid introduced into the drainage opening is received within the receiving channel and impinges upon the pressure sensing mechanism.
2. The assembly of claim 1 wherein the catheter comprises a Foley type catheter.
3. The assembly of claim 1 further comprising an adapter configured for attachment to a proximal end of the catheter, where the port is fluidly coupled to the adapter.
4. The assembly of claim 1 wherein the port is configured to fluidly couple to the drainage lumen along a length of the drainage lumen.
5. The assembly of claim 1 wherein the port is configured to fluidly couple to a proximal end of the drainage lumen.
6. The assembly of claim 1 wherein the fluid chamber is configured to be located external to a patient body.
7. The assembly of claim 1 wherein a proximal end of the drainage lumen is configured to be periodically obstructed.
8. The assembly of claim 1 wherein the pressure sensing mechanism further comprises a pressure sensor attached via a pressure line.
9. The assembly of claim 1 wherein the pressure sensor comprises a mechanical or fiber-optic pressure sensor.
10. The assembly of claim 1 wherein the pressure sensing mechanism comprises a pressure sensing balloon.
11. The assembly of claim 1 wherein the pressure sensing mechanism is configured to transduce pressure impinging on it into a chronological pressure profile, the pressure profile having sufficient resolution to be processed into one or more distinct physiologic pressure profiles, said physiologic pressure profiles selected from a group consisting of peritoneal pressure, respiratory rate, and cardiac rate.
12. The assembly of claim 11 wherein the pressure profile has sufficient resolution such that, when sampled by a transducer at a frequency of at least about 1 Hz, it can be processed to yield a relative pulmonary tidal volume profile.
13. The assembly of claim 11 wherein the pressure profile has sufficient resolution such that, when sampled by a transducer at a frequency of at least about 5 Hz, it can be processed to yield physiologic pressure profiles selected from a group consisting of cardiac output, relative cardiac output, and absolute cardiac stroke volume.
14. The assembly of claim 1 further comprising an analyte sensor in fluid communication with the receiving channel.
15. The assembly of claim 14 wherein the analyte sensor is configured to sense an analyte selected from a group consisting of pH, a gas, an electrolyte, a metabolic substrate, a metabolite, an enzyme, and a hormone.
16. The assembly of claim 1 further comprising one or more electrical activity sensors.
17. The assembly of claim 1 further comprising a light source and a light sensor, the sensor configured to capture light emitted from the light source.
18. A method of sensing fluid pressure, comprising:
   positioning a catheter within a body lumen, the catheter having a length and an expandable retention member located near or at a distal end of the catheter, the catheter defining a drainage lumen at least partially through the catheter length such that a distal end of the drainage lumen terminates at a drainage opening defined near or at the distal end of the catheter;
   introducing a fluid from the body lumen through the drainage opening and into the drainage lumen;
   receiving the fluid through a port fluidly coupled to the drainage lumen and into a receiving channel of a fluid chamber which is positioned external to the body lumen; and
   detecting a fluid pressure from the fluid impinging upon a pressure sensing mechanism located within the fluid chamber.
19. The method of claim 18 wherein the catheter comprises a Foley type catheter.
20. The method of claim 18 wherein receiving the fluid comprises receiving the fluid through the port which is fluid coupled to an adapter configured for attachment to a proximal end of the catheter.
21. The method of claim 18 wherein receiving the fluid comprises fluidly coupling the port to a proximal end of the drainage lumen.
22. The method of claim 18 further comprising periodically stopping fluid flow through the drainage lumen.
23. The method of claim 18 wherein detecting a fluid pressure comprises sensing the fluid pressure via a pressure sensor attached via a pressure line.
24. The method of claim 18 wherein the pressure sensing mechanism comprises a pressure sensing balloon.
25. The method of claim 18 further comprising transducing the fluid pressure impinging upon the pressure sensing mechanism into a chronological pressure profile, the pressure profile having sufficient resolution to be processed into one or more distinct physiologic pressure profiles, said physiologic pressure profiles selected from a group consisting of peritoneal pressure, respiratory rate, and cardiac rate.
26. The method of claim 25 wherein the pressure profile has sufficient resolution such that, when sampled by a transducer at a frequency of at least about 1 Hz, it can be processed to yield a relative pulmonary tidal volume profile.
27. The method of claim 25 wherein the pressure profile has sufficient resolution such that, when sampled by a transducer at a frequency of at least about 5 Hz, it can be processed to yield physiologic pressure profiles selected from a group consisting of cardiac output, relative cardiac output, and absolute cardiac stroke volume.
28. The method of claim 18 further comprising sensing an analyte in the fluid via an analyte sensor in fluid communication with the receiving channel.
29. The method of claim 28 wherein the analyte sensor is configured to sense an analyte selected from a group consisting of pH, a gas, an electrolyte, a metabolic substrate, a metabolite, an enzyme, and a hormone.
30. A fluid pressure sensing apparatus, comprising:
   a catheter having a length and an expandable retention member located near or at a distal end of the catheter, the catheter defining a drainage lumen at least partially through the catheter length such that a distal end of the drainage lumen terminates at a drainage opening defined near or at the distal end of the catheter; and
   a pressure sensing catheter having a pressure sensing mechanism located near or at a distal end of the pressure sensing catheter, wherein the pressure sensing catheter has a diameter sized for insertion within the drainage lumen.
31. The apparatus of claim 30 wherein the pressure sensing catheter has a diameter of 1 mm to 4 mm.
32. The apparatus of claim 30 wherein the pressure sensing mechanism has an expanded diameter of 1 mm to 5 mm.
33. The apparatus of claim 30 wherein the catheter comprises a Foley type catheter.
34. The apparatus of claim 30 wherein the pressure sensing mechanism comprises a pressure sensing balloon.
35. The apparatus of claim 30 wherein the pressure sensing mechanism is configured to transduce pressure impinging on it into a chronological pressure profile, the pressure profile having sufficient resolution to be processed into one or more distinct physiologic pressure profiles, said physiologic pressure profiles selected from a group consisting of peritoneal pressure, respiratory rate, and cardiac rate.
36. The apparatus of claim 35 wherein the pressure profile has sufficient resolution such that, when sampled by a transducer at a frequency of at least about 1 Hz, it can be processed to yield a relative pulmonary tidal volume profile.
37. The apparatus of claim 35 wherein the pressure profile has sufficient resolution such that, when sampled by a transducer at a frequency of at least about 5 Hz, it can be processed to yield physiologic pressure profiles selected from a group consisting of cardiac output, relative cardiac output, and absolute cardiac stroke volume.
38. A method of sensing fluid pressure, comprising:
   positioning a catheter within a body lumen, the catheter having a length and an expandable retention member located near or at a distal end of the catheter, the catheter defining a drainage lumen at least partially through the catheter length such that a distal end of the drainage lumen terminates at a drainage opening defined near or at the distal end of the catheter;
   positioning a pressure sensing catheter within the drainage lumen, the pressure sensing catheter having a pressure sensing mechanism located near or at a distal end of the pressure sensing catheter, wherein the pressure sensing catheter has a diameter sized for insertion within the drainage lumen
   introducing a fluid from the body lumen through the drainage opening and into the drainage lumen; and
   detecting a fluid pressure from the fluid impinging upon the pressure sensing mechanism positioned within the drainage lumen.
39. The method of claim 38 wherein the catheter comprises a Foley type catheter.
40. The method of claim 38 further comprising periodically stopping fluid flow through the drainage lumen.
41. The method of claim 38 wherein the pressure sensing mechanism comprises a pressure sensing balloon.
42. The method of claim 38 further comprising transducing the fluid pressure impinging upon the pressure sensing mechanism into a chronological pressure profile, the pressure profile having sufficient resolution to be processed into one or more distinct physiologic pressure profiles, said physiologic pressure profiles selected from a group consisting of peritoneal pressure, respiratory rate, and cardiac rate.
43. The method of claim 42 wherein the pressure profile has sufficient resolution such that, when sampled by a transducer at a frequency of at least about 1 Hz, it can be processed to yield a relative pulmonary tidal volume profile.
44. The method of claim 42 wherein the pressure profile has sufficient resolution such that, when sampled by a transducer at a frequency of at least about 5 Hz, it can be processed to yield physiologic pressure profiles selected from a group consisting of cardiac output, relative cardiac output, and absolute cardiac stroke volume.
45. The method of claim 38 further comprising sensing an analyte in the fluid via an analyte sensor in fluid communication with the drainage lumen.
46. The method of claim 45 wherein the analyte sensor is configured to sense an analyte selected from a group consisting of pH, a gas, an electrolyte, a metabolic substrate, a metabolite, an enzyme, and a hormone.

## Claims

1. A fluid pressure sensing assembly, comprising:
a catheter having a length and an expandable retention member located near or at a distal end of the catheter, the catheter defining a drainage lumen at least partially through the catheter length such that a distal end of the drainage lumen terminates at a drainage opening defined near or at the distal end of the catheter;
a drainage tube and a receptacle fluidly coupled to the drainage lumen such that the drainage tube is in fluid communication with the drainage opening, wherein a fluid is received into the drainage opening and through the drainage lumen;
a pressure sensing mechanism located near or at the distal end of the catheter, wherein the pressure sensing mechanism produces a signal in response to changes in pressure exerted on the pressure sensing mechanism; and
a venting mechanism which is in communication with the drainage tube; and
a pump in communication with the drainage tube and which is configured to form a negative pressure exerted periodically on the fluid in the drainage tube such that a flow of the fluid through the drainage tube is unobstructed.

2. The assembly of claim 1 wherein the catheter comprises a Foley type catheter.

3. The assembly of claim 1 further comprising an adapter configured for attachment to a proximal end of the catheter, where a port is fluidly coupled to the adapter.

4. The assembly of claim 3 wherein the port is configured to fluidly couple to a proximal end of the drainage lumen.

5. The assembly of claim 1 wherein the pressure sensing mechanism further comprises a pressure sensor attached via a pressure line.

6. The assembly of claim 1 wherein the pressure sensing mechanism comprises a pressure sensing balloon.

7. The assembly of claim 1 wherein the pressure sensing mechanism is configured to transduce pressure impinging on it into a chronological pressure profile, the pressure profile having sufficient resolution to be processed into one or more distinct physiologic pressure profiles, said physiologic pressure profiles selected from a group consisting of respiratory rate, and cardiac rate.

8. The assembly of claim 1 further comprising an analyte sensor.

9. The assembly of claim 8 wherein the analyte sensor is configured to sense bacteria.

10. The assembly of claim 1 further comprising one or more electrical activity sensors.

11. The assembly of claim 1 further comprising a light source and a light sensor, the sensor configured to capture light emitted from the light source.

12. The assembly of claim 1 wherein an inner diameter of the drainage tube is less than or equal to about 6.35 mm.

13. The assembly of claim 1 wherein an inner diameter of the drainage tube is less than or equal to about 3.18 mm.

14. The assembly of claim 1 further comprising a controller configured to determine an intra-abdominal pressure based in part upon changes in pressure sensed by the pressure sensing mechanism.

15. The assembly of claim 14 wherein the controller is configured to store patient data.
